(19) Europäisches Patentamt
European Patent Office
Office européen des brevets

(11) Veröffentlichungsnummer: **0 219 798**
**A1**

(12) **EUROPÄISCHE PATENTANMELDUNG**

(21) Anmeldenummer: **86114184.4**

(22) Anmeldetag: **14.10.86**

(51) Int. Cl.4: **C 07 D 233/90**
C 07 D 233/84, C 07 D 405/12
C 07 D 401/06, C 07 D 403/04
C 07 D 403/06, C 07 D 403/12
C 07 D 413/06, C 07 D 417/04
C 07 D 417/06, A 01 N 43/50

(30) Priorität: **19.10.85 DE 3537290**

(43) Veröffentlichungstag der Anmeldung:
**29.04.87 Patentblatt 87/18**

(84) Benannte Vertragsstaaten:
**AT BE CH DE ES FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Schmierer, Roland, Dr.**
**An der Weinleite 5a**
**D-8901 Todtenweis(DE)**

(72) Erfinder: **Mildenberger, Hilmar, Dr.**
**Fasanenstrasse 24**
**D-6233 Kelkheim (Taunus)(DE)**

(72) Erfinder: **Handte, Reinhard, Dr.**
**Theilweg 23**
**D-8901 Gablingen(DE)**

(72) Erfinder: **Bürstell, Helmut, Dr.**
**Am Hohlacker 65**
**D-6000 Frankfurt am Main 50(DE)**

(54) **1,2,5-substituierte Imidazolverbindungen, Verfahren zu ihrer Herstellung und ihre Verwendung als Wachstumsregulatoren.**

(57) Die Verbindungen der Formel I

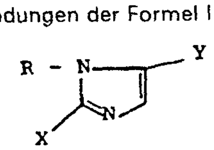

I

worin
R = (subst.) Phenyl, (subst.) Cyclohexyl oder (subst.) Cyclohexenyl

X = einen Rest der Formeln
-SH, $-S-S-R^1$,

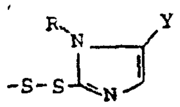

, $-S(O)_m-R^4$ oder $-S-R^5$,

Y = einen Rest der Formeln

$-CN$, $-\overset{O}{\overset{\|}{C}}-OR^6$, $-\overset{O}{\overset{\|}{C}}-SR^7$, $-\overset{S}{\overset{\|}{C}}-OR^7$, $-\overset{S}{\overset{\|}{C}}-SR^7$,

$-\overset{O}{\overset{\|}{C}}-NR^8R^9$, $-\overset{S}{\overset{\|}{C}}-N(R^8)_2$, $-\overset{NOR^8}{\overset{\|}{C}}-NH_2$, $-\overset{O}{\overset{\|}{C}}-R^8$, $-\overset{NOR^{10}}{\overset{\|}{C}}-R^8$, $-CH_2-OR^{10}$,

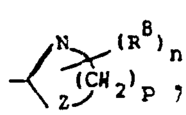

,

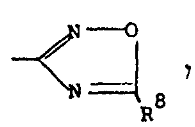

,

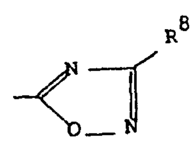

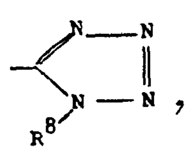

,

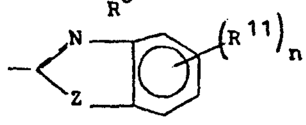

.

./...

EP 0 219 798 A1

sowie die von $-\overset{\displaystyle O}{\overset{\|}{C}}-R^8$
abgeleiteten Bisulfitaddukte, Acetale, Ketale, Thioacetale oder Thioketale,

Z = O, S oder $N-R^8$; m - 0, 1 oder 2; n = 0, 1, 2, 3 oder 4 und p = 2 oder 3 bedeuten,
sowie deren für landwirtschaftliche Zwecke verträglichen Salze und Quaternisierungsprodukte besitzen vorteilhafte pflanzenwachstumsregulierende Wirkungen.

# 1,2,5-substituierte Imidazolverbindungen, Verfahren zu ihrer Herstellung und ihre Verwendung als Wachstumsregulatoren

Es ist bekannt, daß 1-Phenyl-imidazol-5-carbonsäurederivate, s. DE-A 32 17 094, pflanzenwachstumsregulierende Eigenschaften besitzen. Überraschenderweise wurde nun gefunden, daß in 2- Stellung des Imidazolrings durch Schwefelderivate substituierte Verbindungen in verschiedenen Kulturen vorteilhafte pflanzenwachstumsregulierende Wirkungen besitzen.

Die vorliegende Erfindung betrifft daher die neuen Verbindungen der Formel I

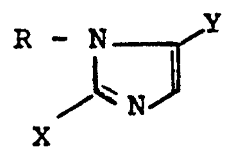

I

worin

R = einen Rest der Formeln

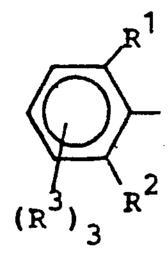

,

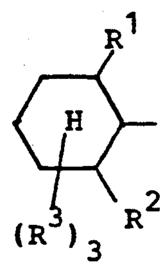

oder

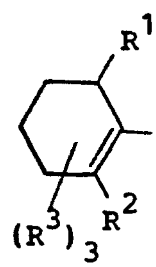

;

X = einen Rest der Formeln -SH, $-S-S-R^1$, 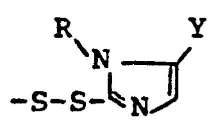 , $-S(O)_m-R^4$ oder $-S-R^5$,

Y = einen Rest der Formeln

$-CN$, $-\overset{O}{\overset{\|}{C}}-OR^6$, $-\overset{O}{\overset{\|}{C}}-SR^7$, $-\overset{S}{\overset{\|}{C}}-OR^7$, $-\overset{S}{\overset{\|}{C}}-SR^7$,

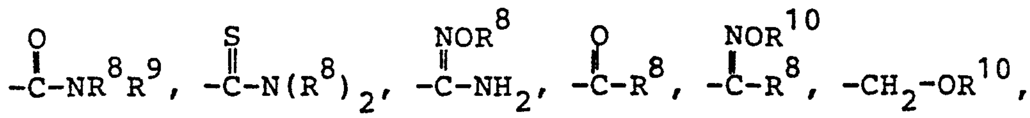

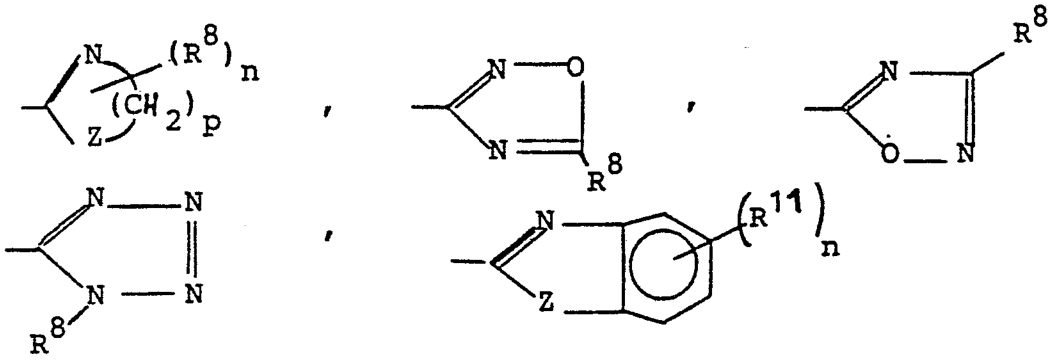

sowie die von $-\overset{O}{\overset{\|}{C}}-R^8$ abgeleiteten Bisulfitaddukte, Acetale, Ketale, Thioacetale oder Thioketale,

Z = O, S oder $N-R^8$

m = 0, 1 oder 2

n = 0, 1, 2, 3 oder 4

p = 2 oder 3

$R^1$, $R^2$ = unabhängig voneinander ($C_1$-$C_4$-Alkyl),

$R^3$ = unabhängig voneinander Wasserstoff, ($C_1$-$C_4$) Alkyl, ($C_1$-$C_4$) Alkoxy oder Halogen,

$R^4$ = ($C_1$-$C_4$)-Alkyl, ($C_2$-$C_4$)-Alkenyl, Propargyl, ($C_1$-$C_4$)Alkoxy-carbonyl-($C_1$-$C_2$)alkyl, Phenyl oder Benzyl,

$R^5$ = ($C_1$-$C_4$)-Alkylcarbonyl, Benzoyl, N-($C_1$-$C_4$)-Alkyl-carbamoyl, ($C_1$-$C_4$)Alkoxy-carbonyl, Phenoxy-carbonyl, ($C_1$-$C_4$)-Alkoxyoxalyl oder, sofern $R^6$ nicht für einen Rest der Formel

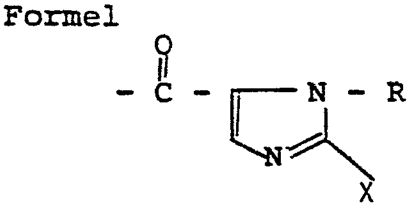

steht, ein für die Landwirtschaft einsetzbares Kation,

$R^6$ = Wasserstoff, $(C_1-C_{12})$Alkyl, $(C_1-C_{12})$-Alkyl, das ein- bis dreifach durch Hydroxy, Halogen, $(C_1-C_6)$Alkoxy, $(C_1-C_4)$Alkoxy-$(C_1-C_4)$alkoxy, $(C_1-C_4)$-Alkylthio, $(C_1-C_4)$Alkylsulfinyl, $(C_1-C_4)$Alkylsulfonyl, Mono- oder Di-$(C_1-C_4$-alkyl$)$amino, Cyano, Aminocarbonyl, $(C_1-C_4)$-Alkanoyl, $(C_1-C_4$-Alkoxy)carbonyl, Cyclo$(C_3-C_7)$-alkyl, Tri$(C_1-C_4$-alkyl)silyl, Benzyloxy, Benzyloxyethoxy, Phenyl oder Phenyl, das durch Halogen oder $(C_1-C_4)$-Alkyl ein- oder mehrfach substituiert ist, durch Phenoxy, Phenylthio, die beide durch Halogen oder $(C_1-C_4)$-Alkyl ein- oder mehrfach substituiert sein können, durch Oxiranyl, Tetrahydrofuryl, Triazolyl, Pyridinyl, Imidazolyl, Carboxy, Carboxylat mit einem für die Landwirtschaft einsetzbaren Kation oder durch den Rest $-O-N=C(CH_3)_2$ substituiert ist,

$(C_3-C_6)$-Alkenyl, halogeniertes $(C_3-C_6)$Alkenyl, unsubstituiertes oder durch Halogen oder $(C_1-C_4)$Alkyl substituiertes Cyclo$(C_3-C_7)$alkyl, unsubstituiertes oder durch Halogen oder $(C_1-C_4)$Alkyl substituiertes Cyclo$(C_5-C_7)$alkenyl, $(C_3-C_6)$Alkinyl, 1,2-Epoxy-prop-3-yl,

Phenyl oder Phenyl, das ein oder zweifach durch Halogen, Nitro, Cyano, $(C_1-C_4)$Alkyl, $(C_1-C_4$-Alkoxy)carbonyl oder $(C_1-C_4)$Alkoxy substituiert ist, $(C_1-C_4$-Alkyl)carbonyl, Phenylcarbonyl, wobei der Phenylring durch Halogen, Nitro, Cyano oder $(C_1-C_4)$-Alkyl substituiert sein kann, einen Rest der Formeln $-N=C(R^{12})_2$, $-NR^8R^{13}$,

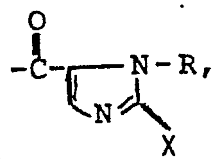

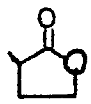 , 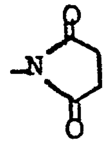 , 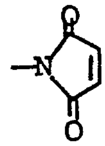 , 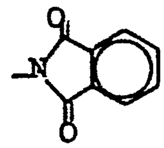

oder ein für die Landwirtschaft einsetzbares Kation,

$R^7$ = H, $(C_1-C_{12})$Alkyl oder $(C_1-C_{12})$Alkyl, das bis zu zweifach durch $(C_1-C_4)$Alkoxy-ethoxy, Cyclo$(C_3-C_6)$ alkyl, Benzyloxy, Phenyl, Phenoxy, $(C_1-C_4)$Alkylthio, $(C_1-C_4$-Alkoxy)-carbonyl, Carboxy oder Carboxylat mit einem für die Landwirtschaft einsetzbaren Kation, substituiert ist, Phenyl oder im Falle $-CS-OR^7$ ein für die Landwirtschaft einsetzbares Kation,

$R^8$ = jeweils unabhängig voneinander Wasserstoff, $(C_1-C_6)$-Alkyl, Phenyl, Benzyl oder Methylphenyl,

$R^9$ = Wasserstoff, $(C_1-C_{12})$Alkyl oder $(C_1-C_{12})$Alkyl, das bis zu zweifach, durch $(C_1-C_6)$Alkoxy, $(C_1-C_4)$-Alkoxy-ethoxy, Hydroxy, Halogen, Cyclo$(C_3-C_6)$alkyl, Benzyloxy, Cyano, Aminocarbonyl, Carboxy, $(C_1-C_4$-Alkoxy)-carbonyl, Formyl, Phenyl oder Phenoxy substituiert ist,

Phenyl oder Phenyl, das bis zu zweifach durch Halogen, Nitro, Cyano, $(C_1-C_4)$-Alkyl oder $(C_1-C_4)$ Alkoxy substituiert ist,

$(C_3-C_6)$-Alkenyl, $(C_3-C_6)$ Cycloalkyl, einen Rest der Formeln $-NR^8R^{14}$, $-OR^8$, $-NH-CO-NH_2$, $-NH-CS-NH_2$ oder $-SO_2R^8$ oder

$R^8$ und $R^9$ gemeinsam mit dem Stickstoffatom an das sie gebunden sind, einen gesättigten oder ungesättigten gegebenenfalls benzokondensierten drei- bis siebengliedrigen Ring, der bis zu drei Heteroatome aus

der Gruppe O, N und S enthält und der unsubstituiert oder durch $(C_1-C_4)$Alkyl oder Halogen substituiert ist und eine Carbonylgruppe enthalten kann,

$R^{10}$ = jeweils unabhängig voneinander H, unsubstituiertes oder durch Phenyl, Halogenphenyl, Nitrophenyl, Cyanophenyl, $(C_1-C_4)$-Alkylphenyl oder $(C_1-C_4)$-Alkoxyphenyl, Hydroxy, Cyano, $(C_1-C_4$-Alkoxy)-carbonyl, $(C_1-C_4)$Alkylthio, $(C_1-C_4)$ Alkoxy, Cyclo$(C_5-C_7)$alkyl oder Benzyloxy substituiertes $(C_1-C_{12})$Alkyl, Cyclo$(C_5-C_8)$alkyl, $(C_3-C_6)$Alkenyl, Halogen$(C_3-C_6)$-alkenyl, $(C_3-C_6)$Alkinyl, Cyclo$(C_5-C_6)$alkenyl, $(C_1-C_6$-Alkyl)carbonyl, Halogen$(C_1-C_6$-alkyl)carbonyl mit 1 bis 3 Halogenatomen, $(C_1-C_6$-Alkyl-amino)carbonyl, Benzoyl, Halogenbenzoyl oder Methylbenzoyl,

$R^{11}$ = jeweils unabhängig voneinander H, Halogen, $(C_1-C_4)$-Alkyl, Nitro oder Cyano,

$R^{12}$ = unabhängig voneinander $(C_1-C_6)$Alkyl, Cyclo$(C_3-C_6)$-alkyl, $(C_3-C_6)$Alkenyl, Phenyl, Benzyl oder beide Reste $R^{12}$ gemeinsam mit dem Kohlenstoffatom, an das sie gebunden sind, ein unsubstituiertes oder durch Methyl oder Halogen substituiertes Cyclo-$(C_5-C_7)$-alkyl,

$R^{13}$ = $(C_1-C_4)$Alkyl, Phenyl, $(C_1-C_6$-Alkyl)carbonyl, Benzyl, Benzoyl, Halogenbenzyl, Halogenbenzoyl oder Methylbenzoyl und

$R^{14}$ = H, $(C_1-C_4)$Alkyl, Formyl, $(C_1-C_6$-Alkyl)carbonyl, Benzoyl, Halogenbenzoyl, Methylbenzoyl oder Trihalogenacetyl, bedeuten

sowie deren für landwirtschaftliche Zwecke verträglichen Salze und Quaternisierungsprodukte.

Die Salzbildung bzw. Quaternisierung erfolgt hierbei durch Addition geeigneter Verbindungen an die $-S-R^4$-Gruppe oder an das basische Stickstoffatom des Imidazolrings. Die Salzbildung oder Quaternisierung ist nicht möglich, wenn $R^5$, $R^6$, $R^7$ ein Kation bedeutet, X für -SH steht oder $R^6$, $R^7$ eine Carboxylatgruppe enthält.

Für den Fall, daß in den aufgeführten Substituenten - zusätzlich zum Imidazolring - weitere basische Stickstoffatome auftreten, ist auch eine mehrfache Salzbildung oder Quaternisierung möglich.

Zur Salzbildung am Stickstoff geeignet sind alle anorganischen oder organischen Säuren, die aufgrund ihres pKs-Wertes zur Salzbildung befähigt sind, z.B. Halogenwasserstoffsäuren, Salpetersäure, Schwefelsäure, Phosphorsäure, Phosphonsäure, Sulfonsäuren, Halogenessigsäuren oder Oxalsäure.

Als Sulfoniumsalze (Salzbildung am Schwefel) oder Quaternisierungsprodukte (Salzbildung am Stickstoff) sind die Umsetzungsprodukte mit Alkyl-, Alkylthioalkyl-, Alkoxy-alkyl-, insbesondere $(C_1-C_6)$Alkyl- und gegebenenfalls im Phenylrest substituierten, insbesondere halogenierten Phenacylhalogeniden zu verstehen. Die Herstellung der Salze und Quaternisierungsprodukte der Verbindungen der Formel I erfolgt nach allgemein üblichen Methoden.

Als Acetale, Ketale und Thioketale kommen insbesondere solche der Formel $-C(OR^{15})_2R^8$ oder $-C(SR^{15})_2R^8$ in Betracht worin $R^{15}$ = $(C_1-C_2)$Alkyl oder beide Reste $R^{15}$ zusammen eine $C_2$ oder $C_3$ Alkylenkette bedeuten, die durch $(C_1-C_4)$ Alkyl, Monohydroxy$(C_1-C_4)$Alkyl, Halogen$(C_1-C_4)$alkyl mit 1 bis 3 Halogenatomen oder Phenyl substituiert sein kann.

Unter den Heterocyclen-Ringen für $-NR^8R^9$ kommen insbesondere infrage Piperidin, Morpholin, 2,6-Dimethylmorpholin, Piperazin, Imidazol, Thiazol oder Benzimidazol.
Die Erfindung umfaßt alle optischen Isomeren der Verbindungen der Formel I. Diese können auftreten, wenn sie asymmetrisch substituierte Cycloalkyl- oder Cycloalkenylringe enthalten. Die bei der Definition der allgemeinen Formel (I) vorkommenden Alkyl-, Alkenyl- und Alkinylreste können sowohl geradkettig als auch verzweigt sein.

Unter Halogen ist F, Cl, Br oder J, insbesondere F, Cl oder Br zu verstehen.

Halogeniertes $(C_3-C_6)$Alkenyl enthält insbesondere 1 bis 3 Chlor oder Fluoratome.
Halogenphenyl, Halogenbenzyl oder Halogenbenzoyl enthalten insbesondere 1 bis 3 Fluor, Chlor oder Bromatome.
Unter Trihalogenacetyl ist insbesondere Trichlor- und Trifluoracetyl zu verstehen.

Als Kationen für $R^5$, $R^6$ oder $R^7$, die für die Landwirtschaft einsetzbar sind kommen Metallkationen z.B. Alkali- oder Erdalkalikationen wie Na, K, Mg oder organische Kationen wie organisches substituiertes Ammonium organisch substituiertes Phosphonium, Sulfonium oder Sulfoxonium oder andere Stickstoff-Kationen in Betracht.

Organisch substituiertes Ammonium bedeutet, primäres, sekundäres, tertiäres, quartäres, aliphatisches, aromatisches oder heteroaromatisches Ammonium, das 1 bis drei N-Atome enthalten kann. Die Stickstoffatome des Amins können hierbei auch Teil eines cyclischen Systems sein.

Als Beispiele für solche Ammoniumsalze seien genannt: Mono-, Di-, Tri-, Tetra [$(C_1-C_6)$Alkyl]ammonium wie Isopropylammonium, Butylammonium, Stearylammonium, Triethylammonium, Mono-, Di-, Tri-[$(C_1-C_4)$alkoxy$(C_1-C_4)$alkyl]ammonium oder Mono-, Di-, Tri-[$(C_1-C_6)$-alkanol]-ammonium wie Methoxyethylammonium, Methoxypropylammonium, Triethanolammonium, Tripropanolammonium, oder Ammoniumverbindungen mit gemischten Resten wie tert.-Butyldiethanolammonium, Triethylbenzylammonium, Hydroxyethyltrimethylammonium, Chlorethyltrimethylammonium; oder Allylammonium, Diallylammonium, Cyclohexylammonium, Menthylammonium, Aminoethylammonium, Ethylendiammonium, Benzhydrylammonium, Pyrrolidinium, Morpholinium, 3-Pyridylammonium, Piperidinium oder Piperazinium, oder ein von einer Aminosäure oder deren Ester abgeleitetes Ammonium wie $NH_3-CH_2-COOCH_3$.

Entsprechend enthalten organisch substituiertes Phosphonium, organisches Sulfonium oder organisches Sulfoxonium aliphatische oder arylaliphatische Reste.

Andere Stickstoff-Kationen sind beispielsweise Hydrazonium, Hydroxylammonium, Guanidinium, Aminoguanidinium oder deren Substitutionsprodukte.

Bevorzugt unter den Verbindungen der Formel I sind solche, worin

R = einen Rest der Formeln

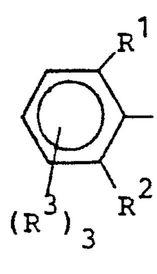 oder 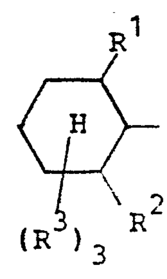

X = einen Rest der Formeln

-SH,

$-S-R^5$, -S-S 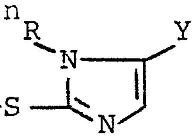 , $-S(O)_m-R^4$ oder

Y = einen Rest der Formeln

$-\overset{O}{\overset{\|}{C}}-OR^6$, $-\overset{O}{\overset{\|}{C}}-NR^8R^9$ oder $-\overset{O}{\overset{\|}{C}}-R^8$

m = 0, 1 oder 2

n = 0, 1, 2 oder 3

$R^1$, $R^2$ = unabhängig voneinander ($C_1$-$C_4$-Alkyl),

$R^3$ = unabhängig voneinander Wasserstoff, ($C_1$-$C_4$)-Alkyl oder Halogen,

$R^4$ = ($C_1$-$C_4$)-Alkyl, ($C_3$-$C_4$)-Alkenyl, Propargyl, ($C_1$-$C_2$)-Alkoxycarbonyl-($C_1$-$C_2$)alkyl, Phenyl oder Benzyl,

$R^5$ = ($C_1$-$C_4$)-Alkylcarbonyl,

$R^6$ = Wasserstoff, ($C_1$-$C_6$)Alkyl, das ein- bis dreifach durch ($C_1$-$C_2$)Alkoxy substituiert ist,

Phenyl, einen Rest der Formeln $-N=C(R^{12})_2$, 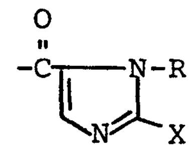 ,

oder ein für die Landwirtschaft einsetzbares Kation,

$R^8$ = jeweils unabhängig voneinander Wasserstoff, ($C_1$-$C_6$)-Alkyl, Phenyl, Benzyl oder Methylphenyl,

$R^9$ = Wasserstoff oder ($C_1$-$C_6$)Alkyl, einen Rest der Formeln $-OR^8$, $-NH-CO-NH_2$ oder $-NH-CS-NH_2$,

$R^{12}$ = unabhängig voneinander ($C_1$-$C_2$)Alkyl bedeuten

sowie deren für landwirtschaftliche Zwecke verträglichen Salze und Quaternisierungsprodukte.

Besonders bevorzugt sind Verbindungen der Formel I, bei denen

R = 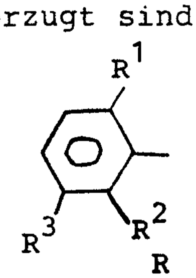 , 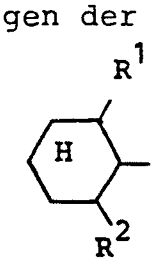

X = -SH, 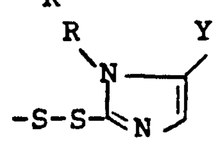 , 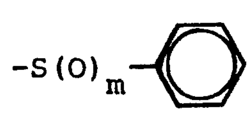 ,

Y = $-\overset{O}{\overset{\|}{C}}-OR^6$, $-\overset{O}{\overset{\|}{C}}-NR^8R^9$, $-\overset{O}{\overset{\|}{C}}-H$,

m = 0, 1 oder 2,

$R^3$ = H oder Halogen,

$R^6$ = H, $C_1$-$C_6$-Alkyl oder $-N=C(CH_3)_2$ oder ein Kation ,

$R^8$, $R^9$ = H, $(C_1$-$C_6)$Alkyl bedeuten.

Gegenstand der Erfindung sind außerdem Verfahren zur Herstellung der Verbindungen der allgemeinen Formel (I);

dadurch gekennzeichnet, daß man

a) einen Bisformylester der Formeln II a oder II b

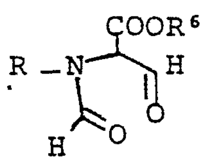
II a

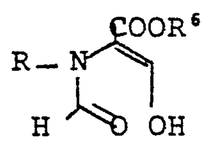
II b

wobei $R^6$ = $(C_1$-$C_{12})$Alkyl bedeutet,

($a_1$) mit einem ($C_1$-$C_3$)Carbonsäureamid cyclisiert oder

($a_2$) mit einem Alkali- oder Ammoniumrhodanid zu einem 2-Mercaptoimidazol der Formel I (X = SH, Y = $-COOR^6$) oder

b) eine Imidazolverbindung der Formel III,

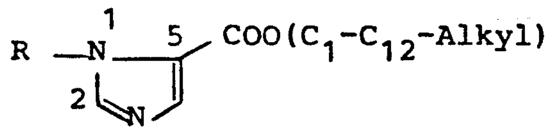

III

mit einer starken Base in 2-Stellung des Imidazolrings metalliert und anschließend mit einem Disulfid der Formel $R^4$-S-S-$R^4$ zu einer Verbindung der Formel I mit X = $-S-R^4$ und Y = COO($C_1$-$C_{12}$-alkyl) umsetzt,

und die unter a) oder b) erhaltenen Verbindungen gegebenenfalls derivatisiert.

Zur Derivatisierung wird der Rest $-COOR^6$ bzw. -COO($C_1$-$C_{12}$-alkyl) in bekannter Weise in andere für Y genannte Reste umgewandelt z.B. durch Verseifung, Veresterung, Umesterung, Amidierung, Salzbildung, Reduktion, Oximierung etc. wie dies z.B. in den deutschen Offenlegungsschriften DE-OS 34 44 918 und DE-OS 34 42 690 beschrieben ist, oder es erfolgt auf übliche Weise Salzbildung oder Quaternisierung am basischen Stickstoffatom des Imidazolringes.

Die Derivatisierung des Restes X, ausgehend von der nach der Variante a) erhaltenen -SH-Verbindung der Formel I erfolgt nach üblichen Verfahren wie z.B. Alkylierung, Acylierung, Carbamoylierung, Salzbildung oder Oxidation.

Die Umwandlung in die Verbindungen der Formel I mit X = $-SR^5$ erfolgt durch Umsetzung mit einem geeigneten Anhydrid, Säurechlorid oder Isocyanat (Houben-Weyl, Methoden der organischen Chemie, Band IX, S. 745 ff) oder mit einer zur Salzbildung befähigten Base.

Bei der Oxidation von Verbindungen der Formel I mit X = SH entstehen zunächst Disulfide mit X = 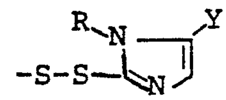

(Houben-Weyl, Band IX S. 59 ff), die durch Umsetzung mit Mercaptanen der Formel $R^1$-SH in Gegenwart von Säuren (Houben-Weyl, Band IX S. 77 ff) zu den Verbindungen der Formel I mit X = $-SSR^1$ umgewandelt werden können.

Die nach Variante b) erhaltenen Verbindungen der Formel I mit X = $S-R^4$ können durch Umsetzung mit einem oder zwei Äquivalenten eines Oxidationsmittels wie m-Chlorperbenzoesäure in die Verbindungen mit X = $-SO-R^4$ bzw. $-SO_2-R^4$ überführt werden. Außerdem kann, falls $R^4$ eine geeignete Schutzgruppe, z.B. $R^4$ = Benzyl, bedeutet, nach Abspaltung von $R^4$ z.B. mit Natrium-Ethanol oder Natrium in Ammoniak auch nach Variante b) eine Mercaptoverbindung der Formel I (X = S-H) erhalten werden, die wie für Variante a) beschrieben, gegebenenfalls weiter derivatisiert werden kann.

Zu a): Im Verfahren ($a_1$) wird als Carbonsäureamid vorzugsweise Formamid eingesetzt. Es wird bevorzugt in Gegenwart von Mineralsäure in molaren Mengen bei 50°-200°C, insbesondere 100°-170°C, umgesetzt.

Die Umsetzung des Bisformylesters II a bzw. II b mit einem Alkali- oder Ammoniumrhodanid wird vorteilhaft in Gegenwart eines Lösungsmittels wie einem Ether z.B. Tetrahydrofuran oder Dioxan oder einem halogenierten Kohlenwasserstoff und molaren Mengen von anorganischen Säuren - zur Freisetzung des Rhodanwasserstoffs - wie z.B. Salzsäure, bei Temperaturen von 0°C bis zum Siedepunkt des Lösungsmittels, vorteilhaft zwischen 20 und 60°C durchgeführt. Das Produkt wird nach beendeter Umsetzung durch Extraktion isoliert und gegebenenfalls chromatographisch gereinigt.

Zu b): Als Basen für die Metallierung sind Natriumamid, Alkyllithiumverbindungen oder Lithiumamide wie z.B. Lithiumdiisopropylamid oder Lithiumcyclohexylisopropylamid in inerten Lösungsmitteln wie Tetrahydrofuran, Hexamethylphosphorsäuretriamid oder 1,3-Dimethylimidazolin-2-on geeignet. Die Temperaturen der Metallierung liegen üblicherweise bei -50 bis -78°C. Nach Zugabe des Disulfids läßt man auf Raumtemperatur erwärmen, hydrolysiert mit Ammoniumchlorid und isoliert das Produkt durch Extraktion. Die Alkylthioverbindungen fallen hierbei in sehr guter Ausbeute und Reinheit an.

Die Bisformylverbindungen der Formel II a bzw. II b lassen sich leicht nach bekannten Verfahren (DE-OS 32 17 094 oder R.G. Jones, J. Am. Chem. Soc., 71, (1949), 644) aus den entsprechenden Anilinen oder Aminen herstellen.

Die Phenylimidazolverbindungen III sind ebenfalls literaturbekannt, s. DE-OS 32 17 094, DE-OS 34 44 918, DE-OS 35 14 116.

Mit den erfindungsgemäßen Verbindungen sind typische wachstumsregulierende Effekte erzielbar, die - verglichen mit den aus der DE-A 32 17 094 bekannten Verbindungen - in verschiedenen Kulturen bereits bei niederen Dosierungen zu beobachten sind. Die Verbindungen der Formel I greifen regulierend in den pflanzeneigenen Stoffwechsel ein und können damit zur gezielten Beeinflussung von Pflanzeninhaltsstoffen sowie zur Ernteerleichterung wie zum Auslösen von Desikkation und Wuchsstauchung eingesetzt werden. Des weiteren eignen sie sich zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern

hierdurch verringert oder völlig verhindert werden kann. Besonders hervorzuheben ist die wachstumsregulatorische Wirksamkeit der Verbindungen als Wuchshemmer in Getreide, Mais, Soja, Tabak, Baumwolle, Ackerbohne, Raps, Reis, Rasen sowie ihre Fähigkeit, den Gehalt an erwünschten Inhaltsstoffen wie Kohlehydrate (z.B. Zuckerrohr oder Hirsekulturen) und Protein bei Nutzpflanzen zu erhöhen. Schließlich zeigen die Verbindungen eine sehr gute Verbesserung der Fruchtabszission, insbesondere bei Zitrusfrüchten.

Ein weiterer Gegenstand der Anmeldung sind auch pflanzenwachstumsregulierende Mittel, die sich durch einen wirksamen Gehalt mindestens einer Verbindung der allgemeinen Formel (I) auszeichnen. Die Aufwandmenge der erfindungsgemäßen Verbindung beträgt im allgemeinen 0,02 bis 1,5 kg Wirksubstanz pro ha, vorzugsweise 0,05 bis 1 kg/ha.

Die erfindungsgemäßen Verbindungen lassen sich bei ihrem praktischen Einsatz gegebenenfalls auch vorteilhaft mit bekannten Wachstumsregulatoren kombinieren. Solche bekannten Wachstumsregulatoren sind die Verbindungen der Formel

$$R\text{-}CH_2\text{-}CH_2\text{-}N^+(CH_3)_3Cl^- \quad \text{(IV)}$$

worin R = OH oder Cl bedeutet (common name Chlormequat für R = Cl), ferner N,N-Dimethylpiperidiniumchlorid (V,Mepiquat-Chlorid, α-Cyclopropyl-4-methoxy-α-(pyrimidin-5-yl)benzylalkohol (VI, Ancymidol), (3aα, 4ß, 4aα, 6aα, 7ß, 7aα)-1-(4-chlorophenyl)-3a, 4, 4a, 6a, 7, 7a-hexahydro-4,7-methano-1H-[1,2]diazeto[3,4-f]benzotriazol (VII, Tetcylacis), Bernsteinsäure-mono-2,2-dimethylhydrazid (VIII, Diaminoazide), 6-Hydroxy-2H-pyridazin-3-on (IX, Maleinsäurehydrazid), 2-Chlor-9-hydroxy-9H-fluoren-9-carbonsäure (X,Chlorflurenol), 5'(Trifluormethansulfonamido)acetat-2',4' -xylidid (XI, Mefluidid), 2-Chlorethylphosphonsäure (XII, Ethephon).

Die wachstumsregulatorischen Wirkungen der Verbindungen der Formeln (IV) bis (XII) sind beschrieben in Plant Growth Regulator Handbook of the Plant Growth Regulator Working Group 2d. Ed.(1981).

Anstelle der Verbindungen der Formeln (VI) und (V) können prinzipiell auch vergleichbare Salze, die anstelle des Chloridions ein anderes übliches Anion wie Bromid, Nitrat oder 1/2 Sulfat enthalten, eingesetzt werden.

Bei der Kombination der Verbindungen der Formel (I) mit den Verbindungen der Formeln (IV) bis (XII) zeigten sich überraschenderweise auffallende synergistische Wirkungen.

...

So lassen sich diese Kombinationen in noch geringeren Dosierungen einsetzten, als von der Wirkung der Einzelkomponenten zu erwarten war, um die gewünschten Effekte zu erzielen. Mit den Kombinationen lassen sich auch Wildwuchserscheinungen vermindern, so daß die Kombinationen auch in der Landschaftspflege eingesetzt werden können. Desweiteren eignen sich die Kombinationen hervorragend zur generellen Steuerung und Hemmung von unerwünschten vegetativen Wachstum, wie Seitentriebbildung, ohne die Pflanzen abzutöten. Die Verbindungen der Formel (I) können auch vorteilhaft mit zwei verschiedenen Verbindungen der Formeln (IV) bis (XII) kombiniert werden.

Die Mischungsverhältnisse der Komponenten der allgemeinen Formel (I) zu den Verbindungen der Formeln (IV) bis (XII) können innerhalb weiter Grenzen, etwa zwischen 250 : 1 bis 1 : 10, schwanken. Die Wahl des Mischungsverhältnisses ist unabhängig von der Art der Mischungspartner, vom Entwicklungsstadium der Pflanzen als auch vom Grad der gewünschten wachstumsregulatorischen Wirkung. Vorzugsweise werden Mischungsverhältnisse von 10 : 1 bis 1 : 10 gewählt.

Die Aufwandmenge der Verbindungen der Formel (I) in den Wirkstoffmischungen liegt im allgemeinen zwischen 0,05 und 1 kg Wirkstoff pro ha, die Aufwandmengen der Verbindungen der Formel (IV) bis (XII) variieren zwischen 0,01 und 5 kg Wirkstoff/ha. Die Kombinationen können sowohl als Mischformulierungen der Komponenten vorliegen, die dann in üblicher Weise mit Wasser verdünnt zur Anwendung gebracht werden; oder sie können als sogenannte Tankmischungen durch gemeinsame Verdünnung der getrennt formulierten Komponenten mit Wasser hergestellt werden; es besteht auch die Möglichkeit, die Komponenten nacheinander zur Anwendung zu bringen, d.h. es werden dann die Komponenten in Einzelformulierungen appliziert.

...

Die Verbindungen der allgemeinen Formel (I) können auch mit natürlichen oder pflanzlichen Hormonen wie Auxinen oder Cytokinen kombiniert werden.

Die erfindungsgemäßen Verbindungen der allgemeinen Formel (I), können, gegebenenfalls im Gemisch mit weiteren Wirkkomponenten, wie den Verbindungen der Formeln II bis XII, als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem (den Wirkstoff(en) außer gegebenenfalls einem Verdünnungs- oder Inertsoff noch Netzmittel wie polyoxethylierte Fettalkohole, Alkyl- oder Alkylphenylsulfonate und/oder Dispergierhilfsmittel wie ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des (der) Wirkstoffe(s) in einem inerten organischen Lösemittel wie Butanol, Cyclohexanon, Dimethylformamid, Xylol oder auch höhersiedenden Aromaten oder aliphatischen oder cycloaliphatischen Kohlenwasserstoffen unter Zusatz von einem oder mehreren Emulgatoren hergestellt werden. Bei flüssigen Wirkstoffen kann der Lösemittelanteil auch ganz oder teilweise entfallen. Als Emulgatoren können beispielsweise verwendet werden: Alkyl-arylsulfonsaure Calciumsalze wie Ca-dodecylbenzolsulfonat oder nichtionische Emulgatoren wie Fettsäurepolyglykolester, Alkylarylpolyglykolether, Fettalkoholpolyglykolether, Propylen-

...

oxid-Ethylenoxid-Kondensationsprodukte, Fettalkohol-Propylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des (der) Wirkstoffe(s) mit feinverteilten, festen Stoffen, z.B. Talkum, natürlichen Tonen wie Kaolin, Bentonit, Pyrophillit oder Diatomeenerde erhalten. Granulate können entweder durch Verdüsen der (des) Wirkstoffe(s) auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 0,025 bis 20 Gew.-% an Wirkstoff(en), versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden. Daneben enthalten die genannten Wirkstoffformulierungen gegebenenfalls die jeweils üblichen Haft-, Netz-, Dispergier-, Emulgier-, Penetrations-, Lösemittel, Füll- oder Trägerstoffe.

...

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen wie Insektiziden, Akariziden, Herbiziden, Dügemitteln oder Fungiziden sind gegebenenfalls möglich.

...

Formulierungsbeispiele

Beispiel 1

Ein Stäubemittel wurde erhalten, indem man a) 10 GT[1)] Wirkstoff(e) und 90 GT Talkum oder einem anderen Inertstoff mischte und in einer Schlagmühle zerkleinerte, oder indem man b) 60 GT Wirkstoff, 35 GT Talkum und 5 GT Haftmittel (z.B. ein Polysaccharid wie ®Rhodopol der Rhone-Poulenc S.A.) in der gleichen Weise homogenisierte.

Beispiel 2

Ein in Wasser leicht dispergierbares, benetzbares Pulver wurde erhalten, indem man 25 GT Wirkstoff(e), 64 GT kaolinhaltigen Quarz als Inertstoff, 10 GT ligninsulfonsaures Kalium und 1 GT oleoylmethyltaurinsaures Natrium als Netz- und Dispergierhilfsmittel mischte und in einer Stiftmühle mahlte. Eine Formulierung mit 5 % Wirkstoffgehalt kann wie folgt zusammengesetzt sein: 5 % Wirkstoff(e), 6 % eines sulfonierten Naphthalinformaldehydkondensats (z.B. ®Dispersogen A der Hoechst AG), 2 % eines Na-Salzes einer Alkylnaphthalinsulfonsäure (z.B. ® Leonil DB der Hoechst AG), 5 % eines Gemisches aus Polypropylenglykol und $SiO_2$ (z.B. ®Acrotin 341 der Hoechst AG), 25 % eines $SiO_2$ (z.B. ®Sipernat der Degussa AG) und 57 % Kaolin Typ 1777.

Beispiel 3

Ein in Wasser leicht dispergierbares Dispersionskonzentrat wurde erhalten indem man 20 GT Wirkstoff(e) mit 6 GT eines Alkylphenolpolyglykolethers (z.B. ®Triton X 207 von Rohm and Haas Co.), 3 GT Isotridecanolpolyglykolether ( 8 Ethylenoxid-Einheiten) und 71 GT paraffinischem Mineralöl (Siedebereich ca. 255 bis über 377 °C) mischte und in einer Reibkugelmühle auf eine Feinheit von unter 5 µm vermahlte.

...

Beispiel 4

Ein emulgierbares Konzentrat wurde erhalten aus 15 GT Wirkstoff(e), 75 GT Cyclohexanon als Lösemittel und 10 GT oxethyliertem Nonylphenol (10 Ethylenoxid-Einheiten) als Emulgator.

1) GT = Gewichtsteile

...

## Chemische Beispiele

### Beispiel 1

**Methyl-1-(2,6-dimethylcyclohexyl)-2-mercapto-imidazol-5-carboxylat**

Zu 29,7 g (0,10 Mol) Methyl-2-(N-formyl-2,6-dimethyl-cyclohexylamino)-3-hydroxy-acrylat in 200 ml Tetrahydrofuran gab man eine Lösung von 19,4 g (0,20 Mol) Kaliumrhodanid in 100 ml Wasser zu. Nach dem Zutropfen von 22 g konz. Salzsäure erhitzte man 12 h auf 60 °C. Nach dem Erkalten trennte man die organische Phase ab und dampfte ein. Nach chromatographischer Reinigung erhielt man 21,3 g (76 % d. Theorie) Methyl-1-(2,6-dimethylcyclohexyl)-2-mercapto-imidazol-5-carboxylat als gelbes Öl.

### Beispiel 2

**Ethyl-2-benzylthio-1-(2,6-diethylphenyl)-imidazol-5-carboxylat**

In einer Lösung von 12,2 g (0,12 Mol) Diisopropylamin und 80 ml (0,13 Mol) 15 % Butyllithiumlösung in Hexan in 100 ml absolutem Tetrahydrofuran tropfte man bei -70 bis -78°C 27,2 g (0,10 Mol) Ethyl-1-(2,6-diethylphenyl)-imidazol-5-carboxylat während 1 Stunde zu. Es wurde 10 min weitergerührt und dann eine Lösung von 32,0 g (0,13 Mol) Dibenzyldisulfid in 50 ml Tetrahydrofuran zugetropft. Man ließ auf Raumtemperatur erwärmen, tropfte 10 ml gesättigte Ammoniumchloridlösung zu, goß auf 500 ml Wasser und extrahierte das Reaktionsgemisch 2 mal mit Toluol. Nach dem Eindampfen und Chromatographieren (Kieselgel, Laufmittel Petroläther (tief) - Essigester 8 : 2) erhielt man 33,5 g (85 % der Theorie) Ethyl-2-benzylthio-1-(2,6-diethylphenyl)-imidazol-5-carboxylat als farbloses Öl.

...

$^1$H-NMR (CDCl$_3$, 60 MHz) = 0,9 - 1,3 (m, 9 H, CH$_3$); 2,15 (q, J = 7Hz, Phenyl-CH$_2$, 4H); 4,01 (q, J = 7 Hz, O-CH$_2$, 2 H); 4,40 (s, 2H, -S-CH$_2$); 7,1-7,4 (m, 8 H, Phenyl-H), 7,87 (s,1H, Imidazol-H)ppm.

## Beispiel 3

**Ethyl-2-benzylsulfinyl-1-(2,6-diethylphenyl)-imidazol-5-carboxylat**

Zu 10 g (0,025 Mol) Ethyl-2-benzylthio-1-(2,6-diethylphenyl)-imidazol-3-carboxylat in 50 ml Chloroform gab man bei Raumtemperatur 5,7 g (0,028 Mol) m-Chlorperbenzoesäure zu. Nach 1 Stunde wurde die abgeschiedene m-Chlorbenzoesäure abgesaugt, die Mutterlauge 2mal mit Hydrogencarbonatlösung gewaschen, über Natriumsulfat getrocknet und eingedampft. Man erhielt in quantitativer Ausbeute Ethyl-2-benzylsulfinyl-1-(2,6-diethylphenyl)-imidazol-5-carboxylat; farbloser Feststoff vom Schmp. 82-83°C.

## Beispiel 4

**Ethyl-2-benzylsulfonyl-1-(2,6-diethylphenyl)-imidazol-5-carboxylat**

Analog zu Beispiel 3 nur unter Verwendung der doppeltmolaren Menge m-Chlorperbenzoesäure erhielt man die Sulfonylverbindung in quantitativer Ausbeute; farbloser Feststoff vom Schmp. 62-66°C.

...

Beispiel 5

2- Benzylthio-1-(2,6-diethylphenyl)-imidazol-5-carbonsäure

31,5 g (0,08 Mol) Ethyl-2-benzylthio-1-(2,6-diethylphenyl)-imidazol-5-carboxylat (vgl. Beispiel 2) wurden mit 30 g 33%-iger Natronlauge bis zur Bildung einer homogenen Lösung bei 80°C gerührt. Man ließ abkühlen, säuerte mit verdünnter Salzsäure auf pH 3 an, saugte das Produkt ab und trocknete im Vakuum. Man erhielt 26,4 g (90 % der Theorie) 2-Benzylthio-1-(2,6-diethylphenyl)-imidazol-5-carbonsäure, ein Feststoff vom Schmp. 152-154°C.

Beispiel 6

1-(2,6-Diethylphenyl)-2-mercapto-imidazol-5-carbonsäure

Zu einer Suspension von 5 g (0,014 Mol) 2-Benzylthio-1-(2,6-diethylphenyl)-imidazol-5-carbonsäure in 50 ml flüssigem Ammoniak gab man bei -40 bis -50°C 2,3 g (0,1 Mol) Natrium portionsweise zu. Nach 1 Stunde gab man bei derselben Temperatur 6 g Ammoniumchlorid zu und ließ über Nacht auf Raumtemperatur erwärmen. Man nahm den Rückstand in 200 ml Wasser auf, säuerte mit halbkonzentrierter Salzsäure auf pH 3 an und saugte ab. Man erhielt 3,0 g (79 % d. Theorie) 1-(2,6-Diethylphenyl)-2-mercaptoimidazol-5-carbonsäure, ein Feststoff mit einem Schmelzpunkt über 230°C.

Beispiel 7 (Salzbildung)

Piperidinium-2-benzylsulfonyl-1-(2,6-diethylphenyl)-imidazol-5-carboxylat

6,5 g (0,016 Mol) 2-Benzylsulfonyl-1-(2,6-diethylphenyl)-imidazol-5-carbonsäure wurden mit 1,6 g (0,019 Mol) Piperidin in 50 ml Methylenchlorid bis zur Auflösung der Carbonsäure

...

gerührt. Es wurde eingedampft, der Rückstand mit Ether zur Kristallisation gebracht und der Feststoff abgesaugt und getrocknet: 6,9 g (87 % der Theorie) Piperidinium-2-benzylsulfonyl-1-(2,6-diethylphenyl)-imidazol-5-carboxylat, ein farbloser Feststoff vom Schmp. 167-170°C.

Weitere Beispiele sind in Tabelle 1 aufgeführt.

Tabelle 1 Imidazolverbindungen der Formel I

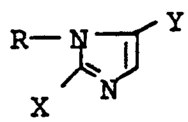

| Bsp.-Nr. | R | X | Y | Fp[°C] |
|---|---|---|---|---|
| 8 | 2,6-Dimethylphenyl | -SH | COOH | Harz |
| 9 | " | " | $COO^{\ominus} \cdot N^{\oplus}(-C_2H_5)_4$ | " |
| 10 | " | $-S-C_4H_9$ | $COO-i-C_3H_7$ | Oel |
| 11 | " | " | CONHNHCHO | " |
| 12 | " | $-S-S-C_2H_5$ | COOH | " |
| 13 | " | $-S-COCH_3$ | CONH-NH-CO-Phenyl | " |
| 14 | " | " | $COOH \cdot NH_3$ | Schaum |
| 15 | " | $-S-COC(CH_3)_3$ | $CONH-NH_2$ | Oel |
| 16 | 2-Ethyl-6-methyl-phenyl | -SH | COOH | " |
| 17 | " | " | $COOCH_3$ | " |
| 18 | " | -SNa | COONa | Harz |
| 19 | " | -SH | COONa | " |
| 20 | " | " | $COOH \cdot H_2NCH_3$ | Oel |
| 21 | " | " | $COO-N{=}C(CH_3)_2$ | " |
| 22 | " | " | $CONHCH_3$ | " |
| 23 | " | " | $CON(CH_3)$Phenyl | " |
| 24 | " | " | $COOCH_2-COOCH_3$ | " |
| 25 | " | " | COOLi | " |
| 26 | " | " | $COO \cdot 1/2Mg$ | Harz |

...

Fortsetzung Tabelle 1

| Bsp.-Nr. | R | X | Y | Fp [°C] |
|---|---|---|---|---|
| 27 | 2-Ethyl-6-methyl-phenyl | -SH | CONHOH | Oel |
| 28 | " | -S-Phenyl | COOH | " |
| 29 | " | -S-Benzyl | COOH | " |
| 30 | " | $-S-CH_2-CH=CH_2$ | COOH | " |
| 1 | " | $-S-CH_2-CH=CH_2$ | $COO-n-C_8H_{17}$ | " |
| 2 | " | $-S-CH_2-COOCH_3$ | $COOC_2H_5$ | " |
| 33 | " | $-S-CH(CH_3)-COO_2H_5$ | $COO-\ C_6H_{11}$ | " |
| 34 | " | $-S-CH_3$ | COO-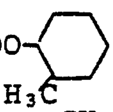 | " |
| 35 | " | $-S-CH_3$ | 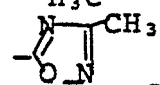 | " |
| 36 | 2,6-Diethyl-phenyl | -SH | COOH·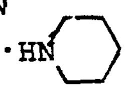 | 194-5 |
| 37 | " | -SH | $COOH\cdot N(-CH_2-CH_2-OH)_3$ | 140-3 |
| 38 | " | -SH | COONa | Schaum |
| 39 | " | -SH | COOK | Harz |
| 40 | " | -SH | $COOH\cdot HN(CH_3)_2$ | Oel |
| 41 | " | -SH | $COOH\cdot N(CH_3)_3$ | " |
| 42 | " | -SH | $COOH\cdot H_2N-CH_2-CH_2-CH_2-OCH_3$ | Oel |
| 43[1)] | " | -SH | COOH | Harz |
| 44[2)] | " | -SH | COOH | " |
| 45 | " | $-S-CH_3$ | COOH | 197-200 |
| 46 | " | $-S-CH_3$ | $COOC_2H_5$ | Oel |
| 47 | " | $-SOCH_3$ | $COOC_2H_5$ | Oel |
| 48 | " | $-SO_2CH_3$ | $COOC_2H_5$ | 119-121 |
| 49 | " | $-S-CH_2-Phenyl$ | $COOH\cdot N(-CH_2-CH_2-OH)_3$ | 80-6 |
| 50 | " | $-SOCH_2-Phenyl$ | CONHOH | Oel |
| 51 | " | $-SO_2-CH_2-Phenyl$ | COOH | 168-171 |

...

Fortsetzung Tabelle 1

| Bsp.-Nr. | R | X | Y | Fp [°C] |
|---|---|---|---|---|
| 52 | 2,6-Diethyl-phenyl | $-SO_2-CH_2-$Phenyl | $COOH \cdot N(-CH_2-CH_2-OH)_3$ | 116-18 |
| 53 | " | 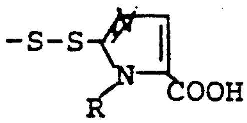 | COOH | Harz |
| 54 | " | 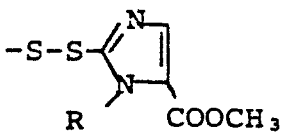 | $COOCH_3$ | Oel |
| 55 | " | -SH | 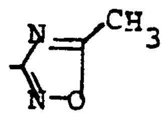 | " |
| 56 | 2,6-Diethyl-3-methyl-phenyl | -S-H | COOH | " |
| 57 | " | $-S-CH_2-$Phenyl | $COOCH_2-COOCH_3$ | " |
| 58 | 2,6-Diethyl-4-methyl-phenyl | -SH | -COOH | " |
| 59 | " | $-S-COOC_2H_5$ | $-COOCH_2-CH_2-O-CH_3$ | " |
| 60 | " | -S-CO-Phenyl | $-CONH-N(CH_3)_2$ | " |
| 61 | " | $-S-CO-NHCH_3$ | $-CONH_2$ | " |
| 62 | 4-Brom-2,6-diethylphenyl | -SH | -COOH | Harz |
| 63 | " | -SH | $-COOCH_2$-4-chlorphenyl | Oel |
| 64 | " | -SH | -COOH· 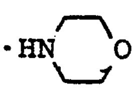 | Oel |
| 65 | 2-Ethyl-6-isopropyl-phenyl | SH | -COOH | Harz |
| 66 | " | $S-COCOOCH_3$ | -COOH | Oel |
| 67 | " | SH | -CO 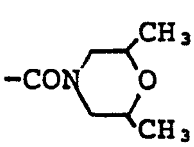 | " |

...

Fortsetzung Tabelle 1

| Bsp.-Nr. | R | X | Y | Fp[°C] |
|---|---|---|---|---|
| 68 | 2-tert.-Butyl-6-methylphenyl | SH | $-COOH$ | Harz |
| 69 | " | SH | $-COCH_3$ | Oel |
| 70 | " | SH | $-C(CH_3)=N-OH$ | " |
| 71 | " | SH | $-C(NH_2)=N-OH$ | " |
| 72 | " | SH | $-CN$ | " |
| 73 | 2-Ethyl-6-methylcyclohexyl | SH | $-COOH$ | " |
| 74 | " | SH | $-CHO$ | " |
| 75 | " | SH | $-CH_2OH$ | " |
| 76 | " | SH | $-CHNOH$ | " |
| 77 | " | SH | $-CHNOCH_3$ | " |
| 78 | 2,6-Diethylcyclohexyl | SH | $-COOH$ | Harz |
| 79 | " | SH | $-COOCH_3$ | Oel |
| 80 | " | SH | $-COOC_2H_5$ | " |
| 81 | " | SH | $-CONH_2$ | " |
| 82 | " | SH | $-CONH$-Phenyl | " |
| 83 | " | SH | $-COO^{\ominus}\cdot N^{\oplus}(CH_2-CH_2-Cl)(CH_3)_3$ | " |
| 84 | 2,6-Diethylcyclohexyl | $-S-CH_3$ | $-COOC_2H_5$ | Oel |
| 85 | " | $-SOCH_3$ | $-COOC_2H_5$ | Oel |

...

Fortsetzung Tabelle 1

| Bsp.-Nr. | R | X | Y | Fp[°C] |
|---|---|---|---|---|
| 86 | 2,6-Diethyl cyclohexyl | $-SO_2-CH_3$ | $-COOC_2H_5$ | Oel |
| 87 | " | $-S-CH_2-$Phenyl | $-COOC_2H_5$ | Oel |
| 88 | " | " | $-COOH$ | 116-24 |
| 89 | " | 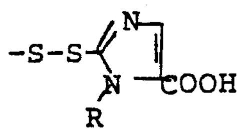 | $-COOH$ | Oel |
| 90 | " | $-S-CH_3$ | 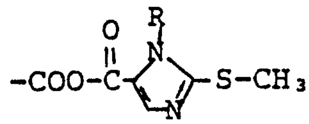 | " |
| 91[3)] | " | $-SH$ | $-COOH$ | Harz |
| 92 | " | $-S^{\oplus}-(CH_3)_3\ J^{\ominus}$ | $-COOC_2H_5$ | Oel |
| 93 | " | $-S-CH_3$ | $-COO-CH_2-COOH$ | " |
| 94 | " | " | 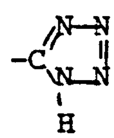 | " |
| 95 | " | " | $-CH(SC_2H_5)_2$ | " |
| 96 | " | " | $-\overset{S}{\overset{\|}{C}}-NH_2$ | " |
| 97 | " | " | 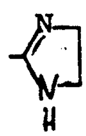 | " |
| 98 | 2,6-Diethylphenyl | -S-Phenyl | COOH | 212-4 |
| 99 | " | " | $COOC_2H_5$ | Öl |
| 100 | " | $-S(O_4)-$Phenyl | " | " |
| 101 | " | $-S(O)_2-$Phenyl | " | " |
| 102 | 2,2-Dimethylcyclo-hexyl | -SH | COOH | Harz |
| 103 | " | " | $COOC_2H_5$ | 119-125 |
| 104 | 2-Ethyl-6-methyl-cyclohexyl | $-SCH_3$ | $COOC_2H_5$ | Öl |
| 105 | 2,6-Diethylcyclo-hexyl | $-S-C(=O)CH_3$ | $COOC_2H_5$ | " |
| 106 | " | $-S-CH_2-$Phenyl | $COOC_2H_5$ | " |

...

Fortsetzung Tabelle 1

| Bsp.-Nr. | R | X | Y | Fp(°C) |
|---|---|---|---|---|
| 107 | " | " | COOH· 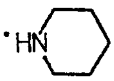 | 109-112 |
| 108 | " | $-SC_2H_5$ | $COOC_2H_5$ | Öl |
| 109 | " | $-S-CH(CH_3)_2$ | " | Öl |
| 110 | " | $-S-CH_2-CH=CH_2$ | " | " |
| 111 | " | $-S-CH_2-C\equiv CH$ | " | " |
| 112 | " | $-S-CH_2-COOC_2H_5$ | " | " |
| 113 | " | $-S-CH_2-COOH$ | COOH | Harz |
| 114 | " | 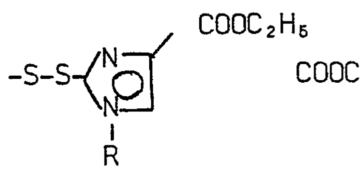 | $COOC_2H_5$ | Öl |
| 115 | 3-Chlor-2,6-diethyl-phenyl | -SH | COOH | Harz |
| 116 | " | " | $COOC_2H_5$ | Öl |
| 117 | " | " | $C(=O)NH_2$ | " |

1) Hydrochlorid

2) Sulfat

3) Chlorethylphosphonat

...

## Biologische Beispiele

### 1. Wuchshemmung an Getreide

In Schalenversuchen im Gewächshaus wurden junge Getreidepflanzen (Weizen, Gerste, Roggen) im 3-Blattstadium mit erfindungsgemäßen Verbindungen in verschiedenen Wirkstoffkonzentrationen (kg/ha) tropfnaß gespritzt.

Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindung beobachtet. Die Wuchshemmung wurde als prozentualer Wert ermittelt, wobei 100 % den Stillstand des Wachstums und 0 % ein Wachstum entsprechend dem der unbehandelten Kontrollpflanzen bedeuten. Es zeigte sich, daß die Verbindungen sehr gute wachstumsregulierende Eigenschaften besitzen , siehe Tabelle 2.

### 2. Wuchshemmung in Wasserreis

Reispflanzen wurden angezogen und im Stadium der maximalen Bestockung mit erfindungsgemäßen Verbindungen behandelt. Die Substanzen wurden sowohl durch Spritzung appliziert als auch in das Wasser gegeben.

3 Wochen nach Behandlung wurde bei allen Pflanzen der Zuwachs gemessen und die Wuchshemmung in % des Zuwachses der Kontrollpflanzen berechnet. Es wurde außerdem auf eine mögliche phytotoxische Wirkung der Verbindungen geachtet.

...

Die Wuchshemmung wurde als prozentualer Wert ermittelt, wobei 100 % den Stillstand des Wachstums und 0 % ein Wachstum entsprechend dem der unbehandelten Kontrollpflanzen bedeuten. Es zeigte sich, daß die Verbindungen sehr gute wachstumsregulierenden Eigenschaften besitzen, siehe Tabelle 3.

3. Wuchshemmung an Sojabohnen

Ca. 10 cm große Sojabohnen wurden mit den Wirkstoffzubereitungen tropfnaß bespritzt. Nach 3 Wochen wurde bonitiert.

Die Wuchshemmung wurde als prozentualer Wert ermittelt, wobei 100 % den Stillstand des Wachstums und 0 % ein Wachstum entsprechend dem der unbehandelten Kontrollpflanzen bedeuten. Es zeigte sich, daß die Verbindungen sehr gute wachstumsregulierenden Eigenschaften besitzen, siehe Tabelle 4.

...

Tabelle 2

| Verb. nach Bsp. Nr. | Anwendungs-konzentr. (kg/ha) | Wuchshemmung in % bei Weizen | Gerste | Roggen | Phytotoxische Wirkung |
|---|---|---|---|---|---|
| 5 | 2.5 | 12 | 15 | 11 | keine Schäden |
| | 1.25 | 8 | 9 | 2 | |
| 6 | 2.5 | 21 | 35 | 15 | |
| | 1.25 | 15 | 20 | 12 | |
| 36 | 2.5 | 22 | 36 | 14 | |
| | 1.25 | 15 | 19 | 12 | |
| 37 | 2.5 | 22 | 35 | 14 | |
| | 1.25 | 14 | 20 | 11 | |
| 45 | 2.5 | 13 | 14 | 10 | |
| | 1.25 | 9 | 7 | 8 | |
| 78 | 2.5 | 12 | 14 | 11 | |
| | 1.25 | 7 | 8 | 6 | |
| 80 | 2.5 | 11 | 15 | 12 | |
| | 1.25 | 9 | 8 | 7 | |
| 99 | 2.5 | 20 | 25 | 21 | |
| | 1.25 | 14 | 19 | 17 | |
| 114 | 2.5 | 21 | 23 | 20 | |
| | 1.25 | 17 | 18 | 16 | |
| A | 2.5 | 27 | 8 | 10 | |
| | 1.25 | 23 | 0 | 0 | |

A = 2-Chlorethyltrimethylammoniumchlorid

...

Tabelle 3

| Verb. nach Bsp. Nr. | Anwendungs-konzentr. (kg/ha) | Wuchshemmung (%) | Phytotoxische Wirkung |
|---|---|---|---|
| 5 | 2.5 | 14 | keine Schäden |
| | 1.25 | 9 | |
| 6 | 2.5 | 17 | |
| | 1.25 | 12 | |
| 36 | 2.5 | 18 | |
| | 1.25 | 11 | |
| 37 | 2.5 | 23 | |
| | 1.25 | 19 | |
| 45 | 2.5 | 15 | |
| | 1.25 | 7 | |
| 78 | 2.5 | 14 | |
| | 1.25 | 11 | |
| 80 | 2.5 | 13 | |
| | 1.25 | 10 | |
| 99 | 2.5 | 7 | |
| | 1.25 | 6 | |
| 114 | 2.5 | 19 | |
| | 1.25 | 17 | |

...

Tabelle 4

| Verb. nach Bsp. Nr. | Anwendungs-konzentr. (kg/ha) | Wuchshemmung (%) | Phytotoxische Wirkung |
|---|---|---|---|
| 5 | 2,5 | 14 | keine Schäden |
| 6 | 2,5 | 21 | |
| 36 | 2,5 | 24 | |
| 37 | 2,5 | 22 | |
| 45 | 2,5 | 11 | |
| 78 | 2,5 | 9 | |
| 80 | 2,5 | 11 | |
| 99 | 2,5 | 16 | |
| 114 | 2,5 | 17 | |

...

Patentansprüche

1. Verbindungen der Formel I

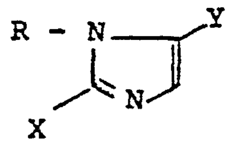 I

worin

R = einen Rest der Formeln

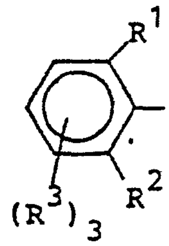 , 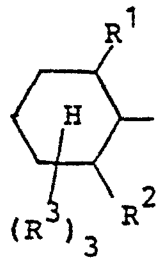 oder 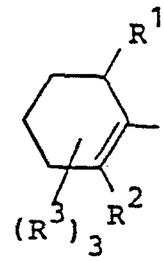 ;

X = einen Rest der Formeln $-SH$, $-S-S-R^1$, 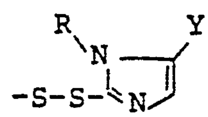 , $-S(O)_m-R^4$ oder $-S-R^5$,

Y = einen Rest der Formeln

$-CN$, $-\overset{O}{\overset{\|}{C}}-OR^6$, $-\overset{O}{\overset{\|}{C}}-SR^7$, $-\overset{S}{\overset{\|}{C}}-OR^7$, $-\overset{S}{\overset{\|}{C}}-SR^7$

$-\overset{O}{\overset{\|}{C}}-NR^8R^9$, $-\overset{S}{\overset{\|}{C}}-N(R^8)_2$, $-\overset{NOR^8}{\overset{\|}{C}}-NH_2$, $-\overset{O}{\overset{\|}{C}}-R^3$, $-\overset{NOR^{10}}{\overset{\|}{C}}-R^8$, $-CH_2-OR^{10}$,

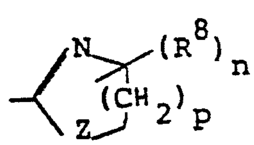 , 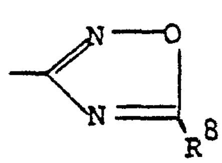 , 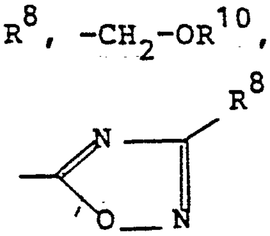

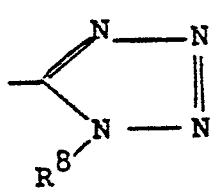 , 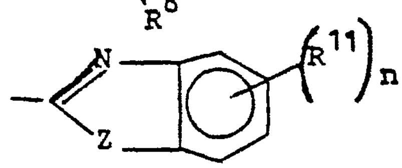

sowie die von $-\overset{O}{\overset{\|}{C}}-R^8$ abgeleiteten Bisulfitaddukte, Acetale, Ketale, Thioacetale oder Thioketale,

Z = O, S oder N-$R^8$

m = 0, 1 oder 2

n = 0, 1, 2, 3 oder 4

p = 2 oder 3

$R^1$, $R^2$ = unabhängig voneinander ($C_1$-$C_4$-Alkyl);

$R^3$ = unabhängig voneinander Wasserstoff, ($C_1$-$C_4$) Alkyl, ($C_1$-$C_4$) Alkoxy oder Halogen,

$R^4$ = ($C_1$-$C_4$)-Alkyl, ($C_2$-$C_4$)-Alkenyl, Propargyl, ($C_1$-$C_4$)Alkoxy-carbonyl-($C_1$-$C_2$)alkyl, Phenyl oder Benzyl;

$R^5$ = ($C_1$-$C_4$)-Alkylcarbonyl, Benzoyl, N-($C_1$-$C_4$)-Alkyl-carbamoyl, ($C_1$-$C_4$)Alkoxy-carbonyl, Phenoxy-carbonyl, ($C_1$-$C_4$)-Alkoxyoxalyl oder, sofern $R^6$ nicht für einen Rest der Formel

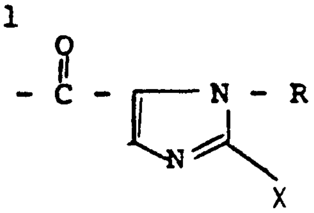

steht, ein für die Landwirtschaft einsetzbares Kation,

$R^6$ = Wasserstoff, ($C_1$-$C_{12}$)Alkyl, ($C_1$-$C_{12}$)-Alkyl, das ein- bis dreifach durch Hydroxy, Halogen, ($C_1$-$C_6$)Alkoxy, ($C_1$-$C_4$)Alkoxy-($C_1$-$C_4$)alkoxy, ($C_1$-$C_4$)-Alkylthio, ($C_1$-$C_4$)Alkylsulfinyl, ($C_1$-$C_4$)Alkylsulfonyl, Mono- oder Di-($C_1$-$C_4$-alkyl)amino, Cyano, Aminocarbonyl, ($C_1$-$C_4$)-Alkanoyl, ($C_1$-$C_4$-Alkoxy)carbonyl, Cyclo($C_3$-$C_7$)-alkyl, Tri($C_1$-$C_4$-alkyl)silyl, Benzyloxy, Benzyloxyethoxy, Phenyl oder Phenyl, das durch Halogen oder ($C_1$-$C_4$)-

Alkyl ein- oder mehrfach substituiert ist, durch Phenoxy, Phenylthio, die beide durch Halogen oder $(C_1-C_4)$-Alkyl ein- oder mehrfach substituiert sein können, durch Oxiranyl, Tetrahydrofuryl, Triazolyl, Pyridinyl, Imidazolyl, Carboxy, Carboxylat mit einem für die Landwirtschaft einsetzbaren Kation oder durch den Rest $-O-N=C(CH_3)_2$ substituiert ist,

$(C_3-C_6)$-Alkenyl, halogeniertes $(C_3-C_6)$Alkenyl, unsubstituiertes oder durch Halogen oder $(C_1-C_4)$Alkyl substituiertes Cyclo$(C_3-C_7)$alkyl, unsubstituiertes oder durch Halogen oder $(C_1-C_4)$Alkyl substituiertes Cyclo$(C_5-C_7)$alkenyl, $(C_3-C_6)$Alkinyl, 1,2-Epoxy-prop-3-yl,

Phenyl oder Phenyl, das ein oder zweifach durch Halogen, Nitro, Cyano, $(C_1-C_4)$Alkyl, $(C_1-C_4$-Alkoxy)carbonyl oder $(C_1-C_4)$Alkoxy substituiert ist, $(C_1-C_4$-Alkyl)carbonyl, Phenylcarbonyl, wobei der Phenylring durch Halogen, Nitro, Cyano oder $(C_1-C_4)$-Alkyl substituiert sein kann, einen Rest der Formeln $-N=C(R^{12})_2$, $-NR^8R^{13}$,

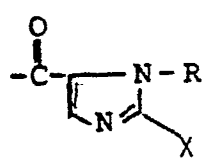,

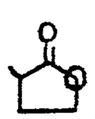 , 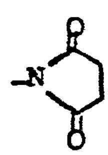 , 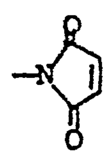 , 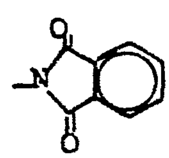 ;

oder ein für die Landwirtschaft einsetzbares Kation;

$R^7$ = H, $(C_1-C_{12})$Alkyl oder $(C_1-C_{12})$Alkyl, das bis zu zweifach durch $(C_1-C_4)$Alkoxy-ethoxy, Cyclo$(C_3-C_6)$alkyl, Benzyloxy, Phenyl, Phenoxy, $(C_1-C_4)$Alkylthio, $(C_1-C_4$-Alkoxy)-carbonyl, Carboxy oder Carboxylat mit einem für die Landwirtschaft einsetzbaren Kation, substituiert ist, Phenyl oder im Falle $-CS-OR^7$ ein für die Landwirtschaft einsetzbares Kation,

$R^8$ = jeweils unabhängig voneinander Wasserstoff, $(C_1-C_6)$-Alkyl, Phenyl, Benzyl oder Methylphenyl,

$R^9$ = Wasserstoff, $(C_1-C_{12})$Alkyl oder $(C_1-C_{12})$Alkyl, das bis zu zweifach, durch $(C_1-C_6)$Alkoxy, $(C_1-C_4)$-Alkoxy-ethoxy, Hydroxy, Halogen, Cyclo$(C_3-C_6)$alkyl, Benzyloxy, Cyano, Aminocarbonyl, Carboxy, $(C_1-C_4$-Alkoxy)-carbonyl, Formyl, Phenyl oder Phenoxy substituiert ist,

Phenyl oder Phenyl, das bis zu zweifach durch Halogen, Nitro, Cyano, $(C_1-C_4)$-Alkyl oder $(C_1-C_4)$ Alkoxy substituiert ist,

$(C_3-C_6)$-Alkenyl, $(C_3-C_6)$ Cycloalkyl, einen Rest der Formeln $-NR^8R^{14}$, $OR^8$, $-NH-CO-NH_2$, $-NH-CS-NH_2$ oder $-SO_2R^8$ oder

$R^8$ und $R^9$ gemeinsam mit dem Stickstoffatom an das sie gebunden sind, einen gesättigten oder ungesättigten gegebenenfalls benzokondensierten drei- bis siebengliedrigen Ring, der bis zu drei Heteroatome aus der Gruppe O, N und S enthält und der unsubstituiert oder durch $(C_1-C_4)$Alkyl oder Halogen substituiert ist und eine Carbonylgruppe enthalten kann;

$R^{10}$ = jeweils unabhängig voneinander H, unsubstituiertes oder durch Phenyl, Halogenphenyl, Nitro-

phenyl, Cyanophenyl, $(C_1-C_4)$-Alkylphenyl oder $(C_1-C_4)$-Alkoxyphenyl, Hydroxy, Cyano, $(C_1-C_4$-Alkoxy)-carbonyl, $(C_1-C_4)$Alkylthio, $(C_1-C_4)$ Alkoxy, Cyclo$(C_5-C_7)$alkyl oder Benzyloxy substituiertes $(C_1-C_{12})$Alkyl, Cyclo$(C_5-C_8)$alkyl, $(C_3-C_6)$Alkenyl, Halogen$(C_3-C_6)$-alkenyl, $(C_3-C_6)$Alkinyl, Cyclo$(C_5-C_6)$alkenyl, $(C_1-C_6$-Alkyl)carbonyl, Halogen$(C_1-C_6$-alkyl)carbonyl mit 1 bis 3 Halogenatomen, $(C_1-C_6$-Alkyl) amino carbonyl, Benzoyl, Halogenbenzoyl oder Methylbenzoyl;

$R^{11}$ = jeweils unabhängig voneinander H, Halogen, $(C_1-C_4)$-Alkyl, Nitro oder Cyano;

$R^{12}$ = unabhängig voneinander $(C_1-C_6)$Alkyl, Cyclo$(C_3-C_6)$-alkyl, $(C_3-C_6)$Alkenyl, Phenyl, Benzyl oder beide Reste $R^{12}$ gemeinsam mit dem Kohlenstoffatom, an das sie gebunden sind, ein unsubstituiertes oder durch Methyl oder Halogen substituiertes Cyclo-$(C_5-C_7)$-alkyl,

$R^{13}$ = $(C_1-C_4)$Alkyl, Phenyl, $(C_1-C_6$-Alkyl)carbonyl, Benzyl, Benzoyl, Halogenbenzyl, Halogenbenzoyl oder Methylbenzoyl und

$R^{14}$ = H, $(C_1-C_4)$Alkyl, Formyl, $(C_1-C_6$-Alkyl)carbonyl, Benzoyl, Halogenbenzoyl, Methylbenzoyl oder Trihalogenacetyl, bedeuten

sowie deren für landwirtschaftliche Zwecke verträglichen Salze und Quaternisierungsprodukte.

2. Verbindungen der Formel I gemäß Anspruch 1, worin

R = einen Rest der Formeln

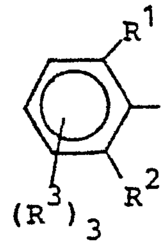

oder

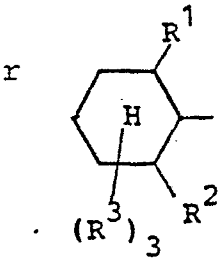

X = einen Rest der Formeln
-SH,
$-S-R^5$, 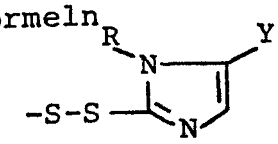 , $-S(O)_m-R^4$ oder

Y = einen Rest der Formeln

$-\overset{O}{\overset{\|}{C}}-OR^6$, $-\overset{O}{\overset{\|}{C}}-NR^8R^9$ oder $-\overset{O}{\overset{\|}{C}}-R^8$

m = 0, 1 oder 2

n = 0, 1, 2 oder 3

$R^1$, $R^2$ = unabhängig voneinander ($C_1$-$C_4$-Alkyl),

$R^3$ = unabhängig voneinander Wasserstoff, ($C_1$-$C_4$)-Alkyl oder Halogen

$R^4$ = ($C_1$-$C_4$)-Alkyl, ($C_3$-$C_4$)-Alkenyl, Propargyl, ($C_1$-$C_2$)-Alkoxycarbonyl-($C_1$-$C_2$)alkyl, Phenyl oder Benzyl,

$R^5$ = ($C_1$-$C_4$)-Alkylcarbonyl,

$R^6$ = Wasserstoff, ($C_1$-$C_6$)Alkyl, das ein- bis dreifach durch ($C_1$-$C_2$)Alkoxy substituiert ist,

Phenyl, einen Rest der Formeln $-N=C(R^{12})_2$, 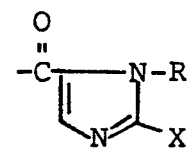 ,

oder ein für die Landwirtschaft einsetzbares Kation,

$R^8$ = jeweils unabhängig voneinander Wasserstoff, ($C_1$-$C_6$)-Alkyl, Phenyl, Benzyl oder Methylphenyl,

$R^9$ = Wasserstoff oder ($C_1$-$C_6$)Alkyl, einen Rest der Formeln $-OR^8$, $-NH-CO-NH_2$ oder $-NH-CS-NH_2$,

$R^{12}$ = unabhängig voneinander ($C_1$-$C_2$)Alkyl bedeuten

sowie deren für landwirtschaftliche Zwecke verträglichen Salze und Quaternisierungsprodukte.

3. Verfahren zur Herstellung der Verbindungen der allgemeinen Formel (I), dadurch gekennzeichnet, daß man

a) einen Bisformylester der Formeln IIa oder IIb

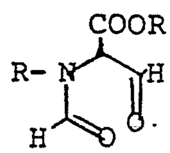

II a

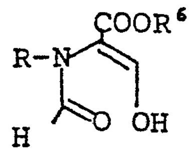

IIb

wobei $R^6$ = $(C_1-C_{12})$Alkyl bedeutet,

($a_1$) mit einem $(C_1-C_3)$Carbonsäureamid cyclisiert oder

($a_2$) mit einem Alkali- oder Ammoniumrhodanid zu einem 2-Mercaptoimidazol der Formel I (X = -SH, Y = $-COOR^6$) oder

b) eine Imidazolverbindung der Formel III,

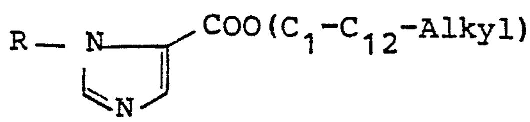

III

mit einer starken Base in 2-Stellung des Imidazolrings metalliert und anschließend mit einem Disulfid der Formel $R^4-S-S-R^4$ zu einer Verbindung der Formel I mit X = $-S-R^4$ und Y = $COO(C_1-C_{12}-alkyl)$ umsetzt,

und die unter a) oder b) erhaltenen Verbindungen gegebenenfalls derivatisiert.

4. Pflanzenwachstumsregulierende Mittel mit einem wirksamen Gehalt an einer Verbindung der allgemeinen Formel (I) von Anspruch 1.

5. Verwendung von Verbindungen der allgemeinen Formel (I) von Anspruch 1 als Wachstumsregulatoren für Pflanzen.

6. Verfahren zur Wachstumsregulierung von Pflanzen, dadurch gekennzeichnet, daß man auf die Pflanzen oder die Anbaufläche eine wirksame Menge einer Verbindung der allgemeinen Formel (I) von Anspruch 1 appliziert.

Patentansprüche Spanien:

Verfahren zur Herstellung der Verbindungen der allgemeinen Formel (I)

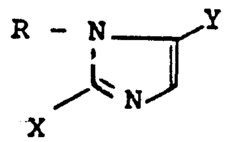 I

worin

R = einen Rest der Formeln

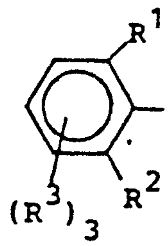 , 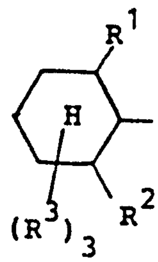 oder 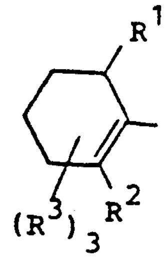 ;

X = einen Rest der Formeln $-SH$, $-S-S-R^1$, $-S-R^5$, 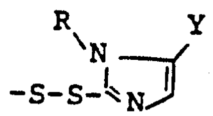 , $-S(O)_m-R^4$ oder

Y = einen Rest der Formeln

$-CN$, $-\overset{O}{\overset{\|}{C}}-OR^6$, $-\overset{O}{\overset{\|}{C}}-SR^7$, $-\overset{S}{\overset{\|}{C}}-OR^7$, $-\overset{S}{\overset{\|}{C}}-SR^7$

$-\overset{O}{\overset{\|}{C}}-NR^8R^9$, $-\overset{S}{\overset{\|}{C}}-N(R^8)_2$, $-\overset{NOR^8}{\overset{\|}{C}}-NH_2$, $-\overset{O}{\overset{\|}{C}}-R^3$, $-\overset{NOR^{10}}{\overset{\|}{C}}-R^8$, $-CH_2-OR^{10}$,

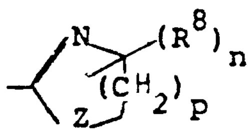 , 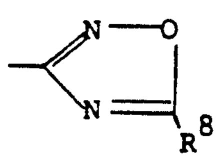 , 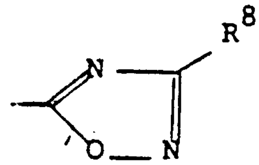

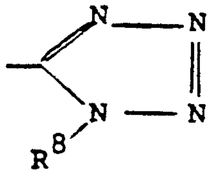 , 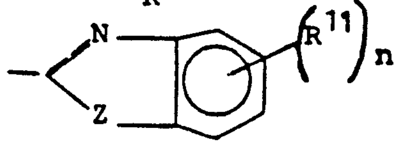

sowie die von $-\overset{O}{\overset{\|}{C}}-R^8$ abgeleiteten Bisulfitaddukte, Acetale, Ketale, Thioacetale oder Thioketale,

Z = O, S oder N-$R^8$

m = 0, 1 oder 2

n = 0, 1, 2, 3 oder 4

p = 2 oder 3

$R^1$, $R^2$ = unabhängig voneinander ($C_1$-$C_4$-Alkyl);

$R^3$ = unabhängig voneinander Wasserstoff, ($C_1$-$C_4$) Alkyl, ($C_1$-$C_4$) Alkoxy oder Halogen,

$R^4$ = ($C_1$-$C_4$)-Alkyl, ($C_2$-$C_4$)-Alkenyl, Propargyl, ($C_1$-$C_4$)Alkoxycarbonyl-($C_1$-$C_2$)alkyl, Phenyl oder Benzyl;

$R^5$ = ($C_1$-$C_4$)-Alkylcarbonyl, Benzoyl, N-($C_1$-$C_4$)-Alkylcarbamoyl, ($C_1$-$C_4$)Alkoxy-carbonyl, Phenoxy-carbonyl, ($C_1$-$C_4$)-Alkoxyoxalyl oder, sofern $R^6$ nicht für einen Rest der Formel

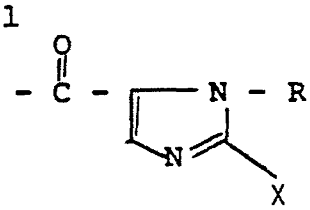

steht, ein für die Landwirtschaft einsetzbares Kation,

$R^6$ = Wasserstoff, ($C_1$-$C_{12}$)Alkyl, ($C_1$-$C_{12}$)-Alkyl, das ein- bis dreifach durch Hydroxy, Halogen, ($C_1$-$C_6$)Alkoxy, ($C_1$-$C_4$)Alkoxy-($C_1$-$C_4$)alkoxy, ($C_1$-$C_4$)-Alkylthio, ($C_1$-$C_4$)Alkylsulfinyl, ($C_1$-$C_4$)Alkylsulfonyl, Mono- oder Di-($C_1$-$C_4$-alkyl)amino, Cyano, Aminocarbonyl, ($C_1$-$C_4$)-Alkanoyl, ($C_1$-$C_4$-Alkoxy)carbonyl, Cyclo($C_3$-$C_7$)-alkyl, Tri($C_1$-$C_4$-alkyl)silyl, Benzyloxy, Benzyloxyethoxy, Phenyl oder Phenyl, das durch Halogen oder ($C_1$-$C_4$)-

Alkyl ein- oder mehrfach substituiert ist, durch Phenoxy, Phenylthio, die beide durch Halogen oder $(C_1-C_4)$-Alkyl ein- oder mehrfach substituiert sein können, durch Oxiranyl, Tetrahydrofuryl, Triazolyl, Pyridinyl, Imidazolyl, Carboxy, Carboxylat mit einem für die Landwirtschaft einsetzbaren Kation oder durch den Rest $-O-N=C(CH_3)_2$ substituiert ist,

$(C_3-C_6)$-Alkenyl, halogeniertes $(C_3-C_6)$Alkenyl, unsubstituiertes oder durch Halogen oder $(C_1-C_4)$Alkyl substituiertes Cyclo$(C_3-C_7)$alkyl, unsubstituiertes oder durch Halogen oder $(C_1-C_4)$Alkyl substituiertes Cyclo$(C_5-C_7)$alkenyl, $(C_3-C_6)$Alkinyl, 1,2-Epoxy-prop-3-yl,

Phenyl oder Phenyl, das ein oder zweifach durch Halogen, Nitro, Cyano, $(C_1-C_4)$Alkyl, $(C_1-C_4$-Alkoxy)carbonyl oder $(C_1-C_4)$Alkoxy substituiert ist, $(C_1-C_4$-Alkyl)carbonyl, Phenylcarbonyl, wobei der Phenylring durch Halogen, Nitro, Cyano oder $(C_1-C_4)$-Alkyl substituiert sein kann, einen Rest der Formeln $-N=C(R^{12})_2$, $-NR^8R^{13}$,

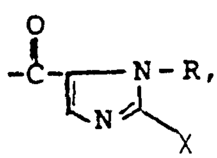

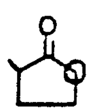 , 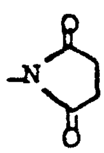 , 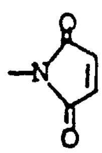 , 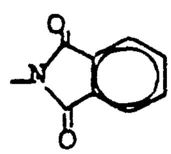 ;

oder ein für die Landwirtschaft einsetzbares Kation;

$R^7$ = H, $(C_1-C_{12})$Alkyl oder $(C_1-C_{12})$Alkyl, das bis zu zweifach durch $(C_1-C_4)$Alkoxy-ethoxy, Cyclo$(C_3-C_6)$ alkyl, Benzyloxy, Phenyl, Phenoxy, $(C_1-C_4)$Alkylthio, $(C_1-C_4$-Alkoxy)-carbonyl, Carboxy oder Carboxylat mit einem für die Landwirtschaft einsetzbaren Kation, substituiert ist, Phenyl oder im Falle $-CS-OR^7$ ein für die Landwirtschaft einsetzbares Kation,

$R^8$ = jeweils unabhängig voneinander Wasserstoff, $(C_1-C_6)$-Alkyl, Phenyl, Benzyl oder Methylphenyl,

$R^9$ = Wasserstoff, $(C_1-C_{12})$Alkyl oder $(C_1-C_{12})$Alkyl, das bis zu zweifach, durch $(C_1-C_6)$Alkoxy, $(C_1-C_4)$-Alkoxy-ethoxy, Hydroxy, Halogen, Cyclo$(C_3-C_6)$alkyl, Benzyloxy, Cyano, Aminocarbonyl, Carboxy, $(C_1-C_4$-Alkoxy)-carbonyl, Formyl, Phenyl oder Phenoxy substituiert ist,

Phenyl oder Phenyl, das bis zu zweifach durch Halogen, Nitro, Cyano, $(C_1-C_4)$-Alkyl oder $(C_1-C_4)$ Alkoxy substituiert ist,

$(C_3-C_6)$-Alkenyl, $(C_3-C_6)$ Cycloalkyl, einen Rest der Formeln $-NR^8R^{14}$, $OR^8$, $-NH-CO-NH_2$, $-NH-CS-NH_2$ oder $-SO_2R^8$ oder

$R^8$ und $R^9$ gemeinsam mit dem Stickstoffatom an das sie gebunden sind, einen gesättigten oder ungesättigten gegebenenfalls benzokondensierten drei- bis siebengliedrigen Ring, der bis zu drei Heteroatome aus der Gruppe O, N und S enthält und der unsubstituiert oder durch $(C_1-C_4)$Alkyl oder Halogen substituiert ist und eine Carbonylgruppe enthalten kann;

$R^{10}$ = jeweils unabhängig voneinander H, unsubstituiertes oder durch Phenyl, Halogenphenyl, Nitro-

phenyl, Cyanophenyl, $(C_1-C_4)$-Alkylphenyl oder $(C_1-C_4)$-Alkoxyphenyl, Hydroxy, Cyano, $(C_1-C_4$-Alkoxy)-carbonyl, $(C_1-C_4)$Alkylthio, $(C_1-C_4)$ Alkoxy, Cyclo$(C_5-C_7)$alkyl oder Benzyloxy substituiertes $(C_1-C_{12})$Alkyl, Cyclo$(C_5-C_8)$alkyl, $(C_3-C_6)$Alkenyl, Halogen$(C_3-C_6)$-alkenyl, $(C_3-C_6)$Alkinyl, Cyclo$(C_5-C_6)$alkenyl, $(C_1-C_6$-Alkyl)carbonyl, Halogen$(C_1-C_6$-alkyl)carbonyl mit 1 bis 3 Halogenatomen, $(C_1-C_6$-Alkyl) amino carbonyl, Benzoyl, Halogenbenzoyl oder Methylbenzoyl;

$R^{11}$ = jeweils unabhängig voneinander H, Halogen, $(C_1-C_4)$-Alkyl, Nitro oder Cyano;

$R^{12}$ = unabhängig voneinander $(C_1-C_6)$Alkyl, Cyclo$(C_3-C_6)$-alkyl, $(C_3-C_6)$Alkenyl, Phenyl, Benzyl oder beide Reste $R^{12}$ gemeinsam mit dem Kohlenstoffatom, an das sie gebunden sind, ein unsubstituiertes oder durch Methyl oder Halogen substituiertes Cyclo-$(C_5-C_7)$-alkyl,

$R^{13}$ = $(C_1-C_4)$Alkyl, Phenyl, $(C_1-C_6$-Alkyl)carbonyl, Benzyl, Benzoyl, Halogenbenzyl, Halogenbenzoyl oder Methylbenzoyl und

$R^{14}$ = H, $(C_1-C_4)$Alkyl, Formyl, $(C_1-C_6$-Alkyl)carbonyl, Benzoyl, Halogenbenzoyl, Methylbenzoyl oder Trihalogenacetyl, bedeuten

sowie deren für landwirtschaftliche Zwecke verträglichen Salze und Quaternisierungsprodukte,

dadurch gekennzeichnet, daß man

a) einen Bisformylester der Formeln IIa oder IIb

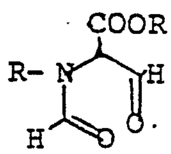

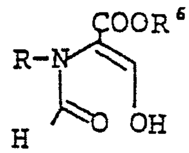

II a IIb

wobei $R^6$ = $(C_1-C_{12})$Alkyl bedeutet,

($a_1$) mit einem $(C_1-C_3)$Carbonsäureamid cyclisiert oder

($a_2$) mit einem Alkali- oder Ammoniumrhodanid zu einem 2-Mercaptoimidazol der Formel I (X = -SH, Y = $-COOR^6$) oder

b) eine Imidazolverbindung der Formel III,

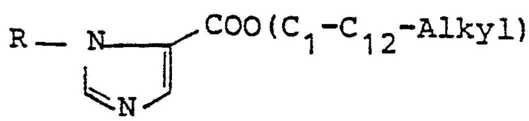

III

mit einer starken Base in 2-Stellung des Imidazolrings metalliert und anschließend mit einem Disulfid der Formel $R^4-S-S-R^4$ zu einer Verbindung der Formel I mit X = $-S-R^4$ und Y = $COO(C_1-C_{12}$-alkyl) umsetzt,

und die unter a) oder b) erhaltenen Verbindungen gegebenenfalls derivatisiert.

2. Verfahren zur Wachstumsregulierung von Pflanzen, dadurch gekennzeichnet, daß man auf die Pflanzen oder die Anbaufläche eine wirksame Menge einer Verbindung der allgemeinen Formel (I) von Anspruch 1 appliziert.

HOE 85/F 231 0219798

Patentansprüche Österreich:

1. Pflanzenwachstumsregulierende Mittel, dadurch gekennzeichnet, daß sie eine Verbindung der Formel I

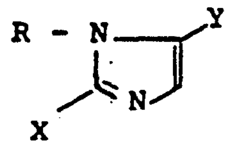 I

worin

R = einen Rest der Formeln

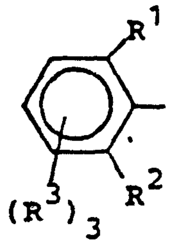 , 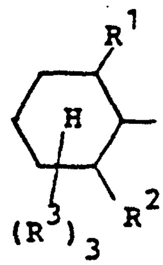 oder 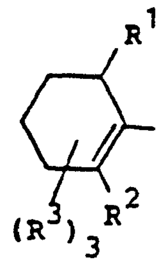 ;

X = einen Rest der Formeln
-SH, $-S-S-R^1$, 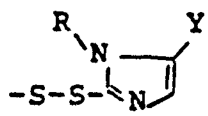 , $-S(O)_m-R^4$ oder $-S-R^5$,

Y = einen Rest der Formeln

$-CN$, $-\overset{O}{\overset{\|}{C}}-OR^6$, $-\overset{O}{\overset{\|}{C}}-SR^7$, $-\overset{S}{\overset{\|}{C}}-OR^7$, $-\overset{S}{\overset{\|}{C}}-SR^7$

$-\overset{O}{\overset{\|}{C}}-NR^8R^9$, $-\overset{S}{\overset{\|}{C}}-N(R^8)_2$, $-\overset{NOR^8}{\overset{\|}{C}}-NH_2$, $-\overset{O}{\overset{\|}{C}}-R^3$, $-\overset{NOR^{10}}{\overset{\|}{C}}-R^8$, $-CH_2-OR^{10}$,

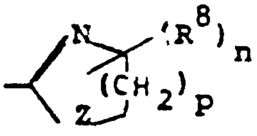 , 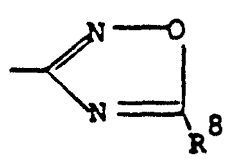 , 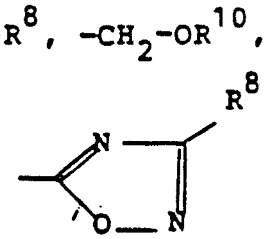

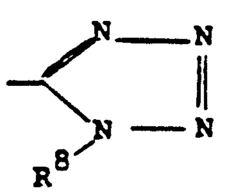 , 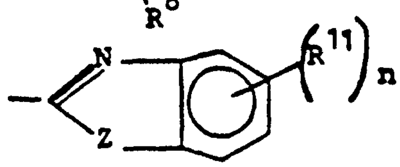

sowie die von $-\overset{O}{\overset{\|}{C}}-R^8$ abgeleiteten Bisulfitaddukte, Acetale, Ketale, Thioacetale oder Thioketale,

Z = O, S oder N-$R^8$

m = 0, 1 oder 2

n = 0, 1, 2, 3 oder 4

p = 2 oder 3

$R^1$, $R^2$ = unabhängig voneinander ($C_1$-$C_4$-Alkyl);

$R^3$ = unabhängig voneinander Wasserstoff, ($C_1$-$C_4$) Alkyl, ($C_1$-$C_4$) Alkoxy oder Halogen,

$R^4$ = ($C_1$-$C_4$)-Alkyl, ($C_2$-$C_4$)-Alkenyl, Propargyl, ($C_1$-$C_4$)Alkoxycarbonyl-($C_1$-$C_2$)alkyl, Phenyl oder Benzyl;

$R^5$ = ($C_1$-$C_4$)-Alkylcarbonyl, Benzoyl, N-($C_1$-$C_4$)-Alkylcarbamoyl, ($C_1$-$C_4$)Alkoxy-carbonyl, Phenoxy-carbonyl, ($C_1$-$C_4$)-Alkoxyoxalyl oder, sofern $R^6$ nicht für einen Rest der Formel

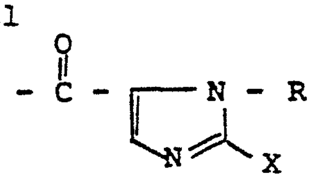

steht, ein für die Landwirtschaft einsetzbares Kation,

$R^6$ = Wasserstoff, ($C_1$-$C_{12}$)Alkyl, ($C_1$-$C_{12}$)-Alkyl, das ein- bis dreifach durch Hydroxy, Halogen, ($C_1$-$C_6$)Alkoxy, ($C_1$-$C_4$)Alkoxy-($C_1$-$C_4$)alkoxy, ($C_1$-$C_4$)-Alkylthio, ($C_1$-$C_4$)Alkylsulfinyl, ($C_1$-$C_4$)Alkylsulfonyl, Mono- oder Di-($C_1$-$C_4$-alkyl]amino, Cyano, Aminocarbonyl, ($C_1$-$C_4$)-Alkanoyl, ($C_1$-$C_4$-Alkoxy)carbonyl, Cyclo($C_3$-$C_7$)-alkyl, Tri($C_1$-$C_4$-alkyl)silyl, Benzyloxy, Benzyloxyethoxy, Phenyl oder Phenyl, das durch Halogen oder ($C_1$-$C_4$)-

Alkyl ein- oder mehrfach substituiert ist, durch Phenoxy, Phenylthio, die beide durch Halogen oder $(C_1-C_4)$-Alkyl ein- oder mehrfach substituiert sein können, durch Oxiranyl, Tetrahydrofuryl, Triazolyl, Pyridinyl, Imidazolyl, Carboxy, Carboxylat mit einem für die Landwirtschaft einsetzbaren Kation oder durch den Rest $-O-N=C(CH_3)_2$ substituiert ist,

$(C_3-C_6)$-Alkenyl, halogeniertes $(C_3-C_6)$Alkenyl, unsubstituiertes oder durch Halogen oder $(C_1-C_4)$Alkyl substituiertes Cyclo$(C_3-C_7)$alkyl, unsubstituiertes oder durch Halogen oder $(C_1-C_4)$Alkyl substituiertes Cyclo$(C_5-C_7)$alkenyl, $(C_3-C_6)$Alkinyl, 1,2-Epoxy-prop-3-yl,

Phenyl oder Phenyl, das ein oder zweifach durch Halogen, Nitro, Cyano, $(C_1-C_4)$Alkyl, $(C_1-C_4$-Alkoxy)carbonyl oder $(C_1-C_4)$Alkoxy substituiert ist, $(C_1-C_4$-Alkyl)carbonyl, Phenylcarbonyl, wobei der Phenylring durch Halogen, Nitro, Cyano oder $(C_1-C_4)$-Alkyl substituiert sein kann,
einen Rest der Formeln $-N=C(R^{12})_2$, $-NR^8R^{13}$,

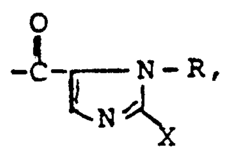

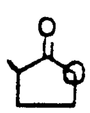 , 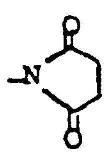 , 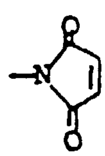 , 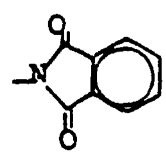 ;

oder ein für die Landwirtschaft einsetzbares Kation;

$R^7$ = H, $(C_1-C_{12})$Alkyl oder $(C_1-C_{12})$Alkyl, das bis zu zweifach durch $(C_1-C_4)$Alkoxy-ethoxy, Cyclo$(C_3-C_6)$alkyl, Benzyloxy, Phenyl, Phenoxy, $(C_1-C_4)$Alkylthio, $(C_1-C_4$-Alkoxy)-carbonyl, Carboxy oder Carboxylat mit einem für die Landwirtschaft einsetzbaren Kation, substituiert ist, Phenyl oder im Falle $-CS-OR^7$ ein für die Landwirtschaft einsetzbares Kation,

$R^8$ = jeweils unabhängig voneinander Wasserstoff, $(C_1-C_6)$-Alkyl, Phenyl, Benzyl oder Methylphenyl,

$R^9$ = Wasserstoff, $(C_1-C_{12})$Alkyl oder $(C_1-C_{12})$Alkyl, das bis zu zweifach, durch $(C_1-C_6)$Alkoxy, $(C_1-C_4)$-Alkoxy-ethoxy, Hydroxy, Halogen, Cyclo$(C_3-C_6)$alkyl, Benzyloxy, Cyano, Aminocarbonyl, Carboxy, $(C_1-C_4$-Alkoxy)-carbonyl, Formyl, Phenyl oder Phenoxy substituiert ist,

Phenyl oder Phenyl, das bis zu zweifach durch Halogen, Nitro, Cyano, $(C_1-C_4)$-Alkyl oder $(C_1-C_4)$ Alkoxy substituiert ist,

$(C_3-C_6)$-Alkenyl, $(C_3-C_6)$ Cycloalkyl, einen Rest der Formeln $-NR^8R^{14}$, $OR^8$, $-NH-CO-NH_2$, $-NH-CS-NH_2$ oder $-SO_2R^8$ oder

$R^8$ und $R^9$ gemeinsam mit dem Stickstoffatom an das sie gebunden sind, einen gesättigten oder ungesättigten gegebenenfalls benzokondensierten drei- bis siebengliedrigen Ring, der bis zu drei Heteroatome aus der Gruppe O, N und S enthält und der unsubstituiert oder durch $(C_1-C_4)$Alkyl oder Halogen substituiert ist und eine Carbonylgruppe enthalten kann;

$R^{10}$ = jeweils unabhängig voneinander H, unsubstituiertes oder durch Phenyl, Halogenphenyl, Nitro-

phenyl, Cyanophenyl, $(C_1-C_4)$-Alkylphenyl oder $(C_1-C_4)$-Alkoxyphenyl, Hydroxy, Cyano, $(C_1-C_4$-Alkoxy)-carbonyl, $(C_1-C_4)$Alkylthio, $(C_1-C_4)$ Alkoxy, Cyclo$(C_5-C_7)$alkyl oder Benzyloxy substituiertes $(C_1-C_{12})$Alkyl, Cyclo$(C_5-C_8)$alkyl, $(C_3-C_6)$Alkenyl, Halogen$(C_3-C_6)$-alkenyl, $(C_3-C_6)$Alkinyl, Cyclo$(C_5-C_6)$alkenyl, $(C_1-C_6$-Alkyl)carbonyl, Halogen$(C_1-C_6$-alkyl)carbonyl mit 1 bis 3 Halogenatomen, $(C_1-C_6$-Alkyl) amino carbonyl, Benzoyl, Halogenbenzoyl oder Methylbenzoyl;

$R^{11}$ = jeweils unabhängig voneinander H, Halogen, $(C_1-C_4)$-Alkyl, Nitro oder Cyano;

$R^{12}$ = unabhängig voneinander $(C_1-C_6)$Alkyl, Cyclo$(C_3-C_6)$-alkyl, $(C_3-C_6)$Alkenyl, Phenyl, Benzyl oder beide Reste $R^{12}$ gemeinsam mit dem Kohlenstoffatom, an das sie gebunden sind, ein unsubstituiertes oder durch Methyl oder Halogen substituiertes Cyclo-$(C_5-C_7)$-alkyl,

$R^{13}$ = $(C_1-C_4)$Alkyl, Phenyl, $(C_1-C_6$-Alkyl)carbonyl, Benzyl, Benzoyl, Halogenbenzyl, Halogenbenzoyl oder Methylbenzoyl und

$R^{14}$ = H, $(C_1-C_4)$Alkyl, Formyl, $(C_1-C_6$-Alkyl)carbonyl, Benzoyl, Halogenbenzoyl, Methylbenzoyl oder Trihalogenacetyl, bedeuten

oder deren für landwirtschaftliche Zwecke verträglichen Salze und Quaternisierungsprodukte enthalten.

2. Pflanzenwachstumsregulierende Mittel gemäß Anspruch 1, dadurch gekennzeichnet, daß sie eine Verbindung der Formel I worin

R = einen Rest der Formeln

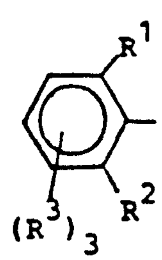

oder

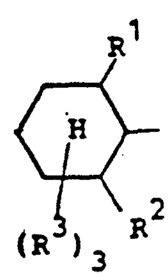

X = einen Rest der Formeln
-SH,
$-S-R^5$, 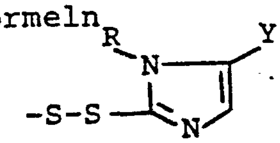 , $-S(O)_m-R^4$ oder

Y = einen Rest der Formeln
$-\overset{O}{\overset{\|}{C}}-OR^6$, $-\overset{O}{\overset{\|}{C}}-NR^8R^9$ oder $-\overset{O}{\overset{\|}{C}}-R^8$

m = 0, 1 oder 2

n = 0, 1, 2 oder 3

$R^1$, $R^2$ = unabhängig voneinander ($C_1$-$C_4$-Alkyl),

$R^3$ = unabhängig voneinander Wasserstoff, ($C_1$-$C_4$)-Alkyl

$R^4$ = ($C_1$-$C_4$)-Alkyl, ($C_3$-$C_4$)-Alkenyl, Propargyl, ($C_1$-$C_2$)-Alkoxycarbonyl-($C_1$-$C_2$)alkyl, Phenyl oder Benzyl,

$R^5$ = ($C_1$-$C_4$)-Alkylcarbonyl,

$R^6$ = Wasserstoff, ($C_1$-$C_6$)Alkyl, das ein- bis dreifach durch ($C_1$-$C_2$)Alkoxy substituiert ist,

Phenyl, einen Rest der Formeln $-N=C(R^{12})_2$, 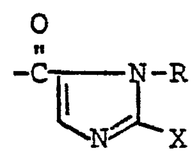 ,

oder ein für die Landwirtschaft einsetzbares Kation,

$R^8$ = jeweils unabhängig voneinander Wasserstoff, ($C_1$-$C_6$)-Alkyl, Phenyl, Benzyl oder Methylphenyl,

$R^9$ = Wasserstoff oder ($C_1$-$C_6$)Alkyl,
einen Rest der Formeln $-OR^8$, $-NH-CO-NH_2$ oder $-NH-CS-NH_2$,

$R^{12}$ = unabhängig voneinander ($C_1$-$C_2$)Alkyl bedeuten

oder deren für landwirtschaftliche Zwecke verträglichen Salze und Quaternisierungsprodukte enthalten.

3. Verfahren zur Herstellung der Verbindungen der allgemeinen Formel (I),
dadurch gekennzeichnet, daß man
a) einen Bisformylester der Formeln IIa oder IIb

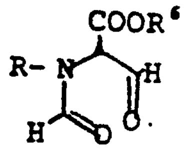

II a

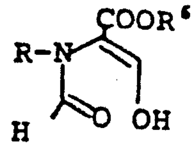

IIb

wobei $R^6$ = $(C_1-C_{12})$Alkyl bedeutet,
($a_1$) mit einem $(C_1-C_3)$Carbonsäureamid cyclisiert oder
($a_2$) mit einem Alkali- oder Ammoniumrhodanid zu einem 2-Mercaptoimidazol der Formel I (X = -SH, Y = $-COOR^6$) oder

b) eine Imidazolverbindung der Formel III,

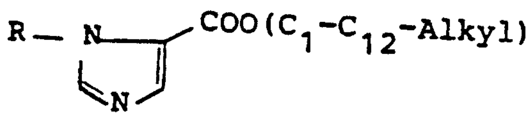

III

mit einer starken Base in 2-Stellung des Imidazolrings metalliert und anschließend mit einem Disulfid der Formel $R^4-S-S-R^4$ zu einer Verbindung der Formel I mit X = $-S-R^4$ und Y = $COO(C_1-C_{12}$-alkyl) umsetzt,
und die unter a) oder b) erhaltenen Verbindungen gegebenenfalls derivatisiert.

4. Verwendung von Verbindungen der allgemeinen Formel (I) von Anspruch 1 als Wachstumsregulatoren für Pflanzen.

5. Verfahren zur Wachstumsregulierung von Pflanzen, dadurch gekennzeichnet, daß man auf die Pflanzen oder die Anbaufläche eine wirksame Menge einer Verbindung der allgemeinen Formel (I) von Anspruch 1 appliziert.

0219798

Europäisches Patentamt

# EUROPÄISCHER RECHERCHENBERICHT

Nummer der Anmeldung

EP 86114184.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 2 732 531 (HOECHST)<br>* Formel IV; Beispiele 5,16; Seiten 26-33 *<br>-- | 1,5 | C 07 D 233/90<br>C 07 D 233/84<br>C 07 D 405/12<br>C 07 D 401/06<br>C 07 D 403/04<br>C 07 D 403/06<br>C 07 D 403/12<br>C 07 D 413/06<br>C 07 D 417/04<br>C 07 D 417/06<br>A 01 N 43/50<br>A 01 N 43/76<br>A 01 N 43/82 |
| D,A | DE - A1 - 3 217 094 (HOECHST)<br>* Zusammenfassung *<br>-- | 1,5 | |
| A | CHEMICAL ABSTRACTS, Band 98, Nr. 7, 14. Februar 1983, Columbus, Ohio, USA<br>BELGODERE; E.; BOSSIO, R.; PARRINI V.; PEPINO, R. "Synthesis of imidazole derivatives with potential biological activity" Seite 661, Spalte 1, Zusammenfassung Nr. 53 763 f<br>& J. Heterocycl. Chem. 1982, 19(3), 561-6<br>-- | 1 | **RECHERCHIERTE SACHGEBIETE (Int. Cl.4)** |
| A | CHEMICAL ABSTRACTS, Band 76, Nr. 25, 19. Juni 1972, Columbus, Ohio, USA<br>GARCIA GONZALEZ, F.; FERNANDEZ BOLANOS, J.; FUENTES MOTA, J. "Thiolglucimidazoles. 9. Synthesis of 3-aryl-(alkyl)4-(D-arabino-tetrahydroxybutyl)imidazoline-2-thiones" Seite 497, Spalte 2, Zusammenfassung Nr. 154 061 b<br>& Carbohyd. Res. 1972, 22(2), 436-40<br>---- | 1 | C 07 D 233/00<br>C 07 D 401/00<br>C 07 D 403/00<br>C 07 D 405/00<br>C 07 D 413/00<br>C 07 D 417/00 |

Der vorliegende Recherchenbericht wurde für alle Patentansprüche erstellt.

| Recherchenort | Abschlußdatum der Recherche | Prüfer |
|---|---|---|
| WIEN | 14-01-1987 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82

Europäisches Patentamt

# EUROPÄISCHER RECHERCHENBERICHT

Nummer der Anmeldung: EP 86114184.4

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| | | | A 01 N 43/74<br>A 01 N 43/64 |
| | | | RECHERCHIERTE SACHGEBIETE (Int. Cl.4) |
| | | | |

Der vorliegende Recherchenbericht wurde für alle Patentansprüche erstellt.

| Recherchenort | Abschlußdatum der Recherche | Prüfer |
|---|---|---|
| WIEN | 14-01-1987 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82